(19) Europäisches Patentamt / European Patent Office / Office européen des brevets

(11) **EP 1 201 753 B1**

(12) **EUROPEAN PATENT SPECIFICATION**


(45) Date of publication and mention of the grant of the patent:
**23.04.2008 Bulletin 2008/17**

(51) Int Cl.:
***C12N 15/10*** *(2006.01)* ***C12Q 1/37*** *(2006.01)*
***C12Q 1/68*** *(2006.01)*

(21) Application number: **01125322.6**

(22) Date of filing: **26.10.2001**


(54) **Methods for the analysis of non-proteinaceous components using a protease from a bacillus strain**

Verfahren zur Analyse von Nicht-Protein Komponenten mittels Verwendung einer Protease aus einem Bacillus-Stamm

Procédé pour l'analyse de composants non-protéiques en utilisant une protéase provenant d'une souche de Bacillus


(84) Designated Contracting States:
**AT BE CH CY DE DK ES FI FR GB GR IE IT LI LU MC NL PT SE TR**

(30) Priority: **31.10.2000 EP 00123728**
**15.03.2001 EP 01106308**

(43) Date of publication of application:
**02.05.2002 Bulletin 2002/18**



(73) Proprietors:
- **Roche Diagnostics GmbH**
  **68305 Mannheim (DE)**
  Designated Contracting States:
  **DE**
- **F. Hoffmann-La Roche AG**
  **4070 Basel (CH)**
  Designated Contracting States:
  **AT BE CH CY DK ES FI FR GB GR IE IT LI LU MC NL PT SE**

(72) Inventors:
- **Russmann, Eberhard, Dr.**
  **82377 Penzberg (DE)**
- **Schmuck, Rainer, Dr.**
  **83671 Benediktbeuern (DE)**
- **Meier, Thomas, Dr.**
  **81369 München (DE)**
- **Staepels, Johnny**
  **82362 Weilheim (DE)**
- **Wehnes, Uwe**
  **68549 Ilvesheim (DE)**



(56) References cited:
**WO-A-89/06279** **WO-A-98/20115**
**DE-A- 4 431 432** **US-A- 4 895 799**

- **R.J.SEIZEN ET AL.: "Subtilases: the superfamily of subtilisin-like serine proteases" PROTEIN SCIENCE, vol. 6, 1997, pages 501-523,**


**EP 1 201 753 B1**


Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

This invention relates to a method for the analysis of a target non-proteinaceous component of a mixture of non-proteinaceous and proteinaceous components derived from a biological sample using a protease from a Bacillus strain. The invention further relates to a method for the analysis of a target nucleic acid component of a mixture of non-proteinaceous components, which comprise nucleic acids, and proteinaceous components whereby the mixture is derived from a biological sample comprising the steps of incubating the mixture with a protease from a Bacillus strain, optionally amplifying the target nucleic acid component, and determining or detecting the target nucleic acid component.

## Background art

Many biological substances, especially nucleic acids, present special challenges in terms of isolating them from their natural environment. On the one hand, they are often present in very small concentrations and, on the other hand, they are often found in the presence of many other solid and dissolved substances e.g. after lysis of cells. This makes them difficult to isolate or to measure, in particular in biospecific assays which allow the detection of specific analytes, e.g. nucleic acids, or specific analyte properties and play a major role in the field of diagnostics and bioanalytics in research and development. Examples for biospecific assays are hybridisation assays, immuno assays and receptor-ligand assays. Hybridisation assays use the specific base-pairing for the molecular detection of nucleic acid analytes e.g. RNA and DNA. Hence, oligonucleotide probes with a length of 18 to 20 nucleotides may enable the specific recognition of a selected complementary sequence e.g. in the human genome. Another assay which entails the selective binding of two oligonucleotide primers is the polymerase chain reaction (PCR) described in US 4,683,195. This method allows the selective amplification of a specific nucleic acid region to detectable levels by a thermostable polymerase in the presence of desoxynucleotide triphosphates in several cycles.

As described above, before the biological substances may be analysed in one of the above-mentioned assays or used for other processes, it has to be isolated or purified from biological samples containing complex mixtures of different components as e.g. proteinaceous and non-proteinaceous components. Often, for the first steps, processes are used which allow the enrichment of the component of interest, e.g. the non-proteinaceous material such as nucleic acids. Frequently, these are contained in a bacterial cell, a fungal cell, a viral particle, or the cell of a more complex organism, such as a human blood cell or a plant cell. The component of interest can also be called a "target component".

To release the contents of said cells or particles, they may be treated with enzymes or with chemicals to dissolve, degrade or denature the cellular walls of such organisms. This process is commonly referred to as lysis. The resulting solution containing such lysed material is referred to as lysate. A problem often encountered during the lysis is that other enzymes degrading the non-proteinaceous component of interest, e.g. desoxyribonucleases or ribonucleases degrading nucleic acids, come into contact with the component of interest during lysis. These degrading enzymes may also be present outside the cells or may have been spatially separated in different cellular compartiments before the lysis and come now into contact with the component of interest. Other components released during this process may be e.g. endotoxins belonging to the family of lipopolysaccharides which are toxic to cells and can cause problems for products intended to be used in human or animal therapy.

There are a variety of means to tackle this problem mentioned-above. It is common to use chaotropic agents as e.g. guanidinium thiocyanate or anionic, cationic, zwitterionic or nonionic detergents when nucleic acids are intended to be set free. It is also an advantage to use proteases which rapidly degrade these enzymes or unwanted proteins. However, this may produce another problem as the said substances or enzymes can interfere with reagents or components in subsequent steps.

Enzymes which can be advantageously used in such lysis or sample preparation processes mentioned-above are enzymes which cleave the amide linkages in protein substrates and which are classified as proteases, or (interchangeably) peptidases (See Walsh, 1979, Enzymatic Reaction Mechanisms. W. H. Freeman and Company, San Francisco, Chapter 3). Proteases which have been used in the prior art are e.g. alkaline proteases (WO98/04730) or acid proteases (US 5,386,024). The protease which is widely used in the prior art for sample preparation for the isolation of nucleic acids is proteinase K from *Tritirachium album* (see e.g. Sambrook et al., 1989) which is active around neutral pH and belongs to a family of proteases known to the person skilled in the art as subtilisins. A subtilisin is a serine protease produced by Gram-positive bacteria or fungi.

Bacteria of the Bacillus species secrete two extracellular species of protease, a neutral or metalloprotease, and an alkaline protease which is functionally a serine endopeptidase, referred to as subtilisin. A serine protease is an enzyme which catalyzes the hydrolysis of peptide bonds, in which there is an essential serine residue at the active site (White, Handler, and Smith, 1973 "Principles of Biochemistry," Fifth Edition, McGraw-Hill Book Company, N.Y., pp. 271-272). The serine proteases have molecular weights in the 25,000 to 30,000 Da (Dalton) range. They hydrolyze simple terminal esters and are similar in activity to eukaryotic chymotrypsin, also a serine protease. The alternative term, alkaline protease, reflects the high pH optimum of the serine proteases, from pH 9.0 to 11.0 (for review, see Priest, 1977, Bacteriological Rev. 41: 711-753).

A wide variety of subtilisins have been identified (see e.g. Kurihara et al., 1972, J. Biol. Chem. 247: 5629-5631; Stahl and Ferrari, 1984, J. Bacteriol. 158: 411-418; Vasantha et al., 1984, J. Bacteriol. 159: 811-819, Jacobs et al., 1985, Nucl. Acids Res. 13: 8913-8926; Nedkov et al., 1985, Biol. Chem. Hoppe-Seyler 366: 421-430; Svendsen et al., 1986, FEBS Lett 196: 228-232;Meloun et al., 1985, FEBS. Lett. 183: 195-200) including proteinase K from Tritirachium album (Jany and Mayer, 1985, Biol. Chem. Hoppe-Seyler 366: 584-492). Subtilisins are well characterized by their primary as well as by their tertiary structure (see e.g. Kraut, 1977, Ann. Rev. Biochem. 46: 331-358; Kurihara et al., 1972, J. Biol. Chem. 247: 5629-5631; Stahl and Ferrari, 1984, J. Bacteriol. 158: 411-418; Vasantha et al., 1984, J. Bacteriol. 159: 811-819; Jacobs et al., 1985, Nucl. Acids Res. 13: 8913-8926; Nedkov et al., 1985, Biol. Chem. Hoppe-Seyler 366: 421-430; Svendsen et al., 1986, FEBS Lett. 196: 228-232; Meloun et al., 1985, FEBS Lett. 183: 195-200; Jany and Mayer, 1985, Biol. Chem. Hoppe-Seyler 366: 485-492).

In connection with this invention the amino acid and DNA sequences of two further serine proteases are of particular interest. These proteases were derived from two Bacillus lentus variants, 147 and 309, which have been deposited with NCIB and designated the accession Nos. NCIB 10147 and NCIB 10309, respectively (see WO89/06279 and US 3,723,250). For convenience the proteases produced by these strains are designated subtilisin 147 and subtilisin 309, respectively, and the genes encoding these proteins are referred to as the subtilisin 147 and 309 genes. The disclosure of these sequences can be found in WO89/06279. The equivalents thereto are EP396608 and US 5,741,694. Subtilisins have found much utility in industry, particularly detergent formulations used for the washing of clothes.

In the next steps of the sample preparation which follow on the lysis step, the component of interest is further enriched. If the non-proteinaceous components of interest are e.g. nucleic acids, they are normally extracted from the complex lysis mixtures before they are used in a probe-based assay.

There are several methods for the extraction of nucleic acids:

- sequence-dependent or biospecific methods as e.g.:
  - affinity chromatography
  - hybridisation to immobilised probes
- sequence-independent or physico-chemical methods as e.g.:
  - liquid-liquid extraction with e.g. phenol-chloroform
  - precipitation with e.g. pure ethanol
  - extraction with filter paper •
  - extraction with micelle-forming agents as cetyl-trimethyl-ammonium-bromide
  - binding to immobilised, intercalating dyes, e.g. acridine derivatives
  - adsorption to silica gel or diatomic earths
  - adsorption to magnetic glass particles (MGP) or organo silane particles under chaotropic conditions

Particularly interesting for extraction purposes is the adsorption of nucleic acids to a glass surface although other surfaces are possible. Many procedures for isolating nucleic acids from their natural environment have been proposed in recent years by the use of their binding behavior to glass surfaces.

As mentioned above, the protease which is widely used in the prior art for sample preparation for the isolation of nucleic acids is proteinase K from *Tritirachium album.* However, this protease has the disadvantage that the production is relatively expensive. Further, proteinase K is disadvantageous in methods using magnetic glass particles for the nucleic acid isolation from EDTA, heparin or citrate blood plasma, as the particles will often stick to one another. This is very disadvantageous for automated processes used for the analysis of a very large number of samples.

Therefore, it was an object of the present invention to provide a new method for the analysis of target non-proteinaceous components, in particular nucleic acids, using a protease which is relatively cheap, has constant quality and can be used in a variety of processes. Preferably it should be possible to use it for the analyis of a (at least one) target nucleic acid component from a variety of different matrices e.g. EDTA, citrate, or heparin blood plasma or blood serum. This method should be particularly suitable in automated processes. Ideally the protease would be also very active in the presence of chaotropic agents frequently used in the processes for the purification of nucleic acids.

This problem was solved by the findings of the present invention which is defined by the appended claims and which is related to a method for the analysis of a (at least one) target non-proteinaceous component of a mixture of non-proteinaceous and proteinaceous components derived from a biological sample comprising the step of incubating the mixture with a (at least one) protease having an amino acid sequence which is at least 80 % identical to the amino acid sequence SEQ ID NO:1 of the protease subtilisin 147 from Bacillus lentus. As can be seen from the example, the protease is very active in the presence of chaotropic agents or equally active for the digestion of citrate or EDTA blood plasma. This could not be foreseen from the prior art.

In summary, this invention relates to a method for the analysis of a (at least one) target non-proteinaceous component of a mixture of non-proteinaceous and proteinaceous components derived from a biological sample using the protease from a Bacillus strain as defined in the appended claims. The invention further relates to a method for the analysis of a (at least one) target nucleic acid component of a mixture of non-proteinaceous components, which comprise nucleic acids, and proteinaceous components whereby the mixture is derived from a biological sample comprising the steps of incubating the mixture with a (at least one) protease having an amino acid sequence which is at least 80 % identical to the amino acid sequence SEQ ID NO:1 of the protease subtilisin 147 from Bacillus lentus, optionally amplifying the (at least one) target nucleic acid component, and determining or detecting the (at least one) target nucleic acid component. Optionally, the nucleic acids and the (at least one) target nucleic acid component are bound to a material with an affinity thereto, optionally washed and optionally released from the material with an affinity thereto, whereby the material with an affinity to nucleic acids and the (at least one) target nucleic acid component comprises a material with a silica surface, in particular magnetic glass particles. The invention is further related to the use of the protease according to the invention in diagnostics, research and bioanalytics e.g. for the purification of nucleic acids, for the analysis of a (at least one) target non-proteinaceous component of a mixture of non-proteinaceous and proteinaceous components derived from a biological sample, for the enrichment of a (at least one) target non-proteinaceous component of a mixture of non-proteinaceous and proteinaceous components derived from a biological sample or for the purification or isolation of a (at least one) target non-proteinaceous component of a mixture of non-proteinaceous and proteinaceous components derived from a biological sample. The invention is also related to a kit comprising the protease according to the invention and the use of a kit according to the invention in diagnostics and/ or for the purification of nucleic acids. The invention will be described in more detail below.

Figure legends:

Figure 1a: Comparison of the digestion of EDTA plasma versus citrate plasma with Esperase as analyzed by high pressure liquid chromatography

Figure 1b: Comparison of the digestion of EDTA plasma versus citrate plasma with proteinase K as analyzed by high pressure liquid chromatography

Figure 2: Determination of the pH Optimum of Esperase

Figure 3: Determination of the residual activity of Esperase versus proteinase K in dependence of the concentration of a chaotropic agent. The highest activity is set to a value of 100 % and the other concentrations are calculated relative to the highest value.

Figure 4: Determination of the stability of Esperase versus proteinase K in dependence from the concentration of guanidinium thiocyanate. The activity of the protease is measured directly after the addition of guanidinium thiocyanate and after 15 min at 25 °C in the presence of guanidinium thiocyanate. The percentage of the residual activity at different guanidinium thiocyanate concentrations is shown in this figure.

Figure 5: Stability in Storage Buffer (composition: 10 mM Tris acetate, 5 mM calcium chloride, 5 mM calcium acetate, 1 mM EDTA, 50 % (V/V) Glycerin with a pH value of 5.5) of esperase versus proteinase K

Description of the invention

It is one embodiment of this invention to provide a method for the analysis of a (at least one) target non-proteinaceous component of a mixture of non-proteinaceous and proteinaceous components derived from a biological sample comprising the step of incubating the mixture with a protease having an amino acid sequence which is at least 80 % identical to the amino acid sequence SEQ ID NO:1. The term "derived" means that a biological sample is manipulated or treated in order to create a mixture of non-proteinaceous and proteinaceous components which are originally contained in the biological sample. From this mixture it should be possible to analyse, isolate, enrich or purify specific non-proteinaceous components. The term "analysis" shall mean that the presence or the amount of the target non-proteinaceous component is investigated, i.e. the target non-proteinaceous component is detected or determined or the amount thereof is determined. Manipulation or treatment steps include chemical or physical manipulation steps which are known to the expert in the field. More specifically, this can be done by lysing the biological sample. Biological samples are samples which are taken from a plant or an animal (including a human being) and are solid or liquid. Specific examples are described in more detail below.

In a further embodiment of the invention, the method has further steps after the incubation as binding the target non-proteinaceous component to a material with an affinity thereto, optionally washing and optionally releasing the target non-proteinaceous component from the material with an affinity thereto. Afterwards, the target non-proteinaceous component may be determined or detected by standard analytical methods known to the person skilled in the art and described e.g. in Sambrook et al. (1989), Molecular Cloning, Cold Spring Harbor University Press, New York, NY, USA or in "Bioanalytik", Lottspeich and Zorbas (eds.), 1st edition 1998, Spektrum Akademischer Verlag, Heidelberg, Berlin, Germany. Preferably, the amount of the target non-proteinaceous component is determined with the methods described therein. The method according to the invention is preferably used in research, bioanalytics in particular in diagnostics or in diagnostic investigations in medicine, i.e. in methods that are used to determine the cause of an illness or disorder in humans or in animals.

Therefore, a preferred embodiment of the invention is a method for the analysis of a target non-proteinaceous component of a mixture of non-proteinaceous and proteinaceous components derived from a biological sample comprising the steps of

a) incubating the mixture with the protease according to the invention,
b) binding the target non-proteinaceous component to a material with an affinity thereto,
c) optionally washing and optionally releasing the target non-proteinaceous component from the material with an affinity thereto, and
d) determining or detecting the target non-proteinaceous component.

In the most preferred embodiment, the step c) is not optional, i.e. that the bound target non-proteinaceous component is washed and released from the material with an affinity thereto. Preferably the amount of the target non-proteinaceous component is determined.

The protease according to the invention degrades the proteinaceous components, i.e. the components containing peptide bonds which shall be hydrolyzed if it is of interest to enrich, isolate or purify the target non-proteinaceous component of the biological sample. The protease according to the present invention may be added in solid form e.g. as a tablet or a powder or in a dissolved form in a buffered or unbuffered solution in a similar manner as described for proteinase K.

For the purpose of this invention, the term "esperase" shall mean the protease according to the invention, i.e. the protease subtilisin 147 derived from the Bacillus lentus variant 147, which was deposited with NCIB under accession No. NCIB 10147. The amino acid sequence SEQ ID NO 1 is the full length amino acid sequence of the protease subtilisin 147 (or esperase) including a signal sequence which is removed after secretion by the action of proteases. A signal sequence is a sequence that directs secretion of an expressed protein from the host cell and is proteolytically removed after secretion. The SEQ ID NO 2 is the sequence of esperase without signal sequence. The term "esperase" shall also comprise those proteolytical derivatives of SEQ ID NO 1 which might be generated by incomplete or inexact processing of the signal sequence and which still have proteolytic activity even those with a lower but still high enough activity. The amino acid sequence of the protein may be encoded by the subtilisin 147 gene, i.e. the nucleotide sequence SEQ ID NO 3, by parts thereof or a degenerated version thereof. Degenerated sequences are degenerated within the meaning of the genetic code in that an unlimited number of nucleotides are replaced by other nucleotides without resulting in a change of the amino acid sequence originally encoded.

According to the present invention the term "proteinaceous material" is meant to describe material that contains a (at least one) peptide bond, therefore "proteinaceous material" is preferably a composition of matter containing a (at least one) protein with natural amino acids. Most of these peptide bonds may be hydrolyzed by the protease according to the present invention depending on the chemical nature of the neighboring chemical groups (or amino acids) and the accessibility of the peptide bond, i.e. the proteinaceous material is a substrate to the protease according to the invention. In consequence, the term "non-proteinaceous material" is meant to describe material that does not contain a peptide bond and is not substrate to the protease according to the present invention.

The protease subtilisin 147 from Bacillus lentus is available to the expert in the field e.g. from Roche Molecular Biochemicals, Mannheim, Germany, or from Novo Nordisk, Denmark. Another possibility to obtain this protease is to isolate the gene from the deposited microorganism or to synthesize the gene coding for that protease according to standard methodology see e.g. Sambrook et al. (1989), Molecular Cloning, Cold Spring Harbor University Press, New York, NY, USA. The amino acid sequence of the pro-protein comprising a signal sequence (SEQ ID NO 1), the amino acid sequence of the secreted protease (SEQ ID NO 2) and the DNA sequence (see SEQ ID NO 3) of this protein are known from WO89/06279, EP 396 608 and WO98/20115. The major form of the secreted protein is encoded by the nucleotides 280 to 1083 of SEQ ID NO 3, i.e. the signal peptide is encoded by the nucleotides 1 to 279 of SEQ ID NO 3. The isolation of the microorganism is described in US 3,723,250. The isolated strain is deposited under NCIB 10147. Custom gene synthesis can be performed by example by Operon Technologies, Alameda, CA, USA, recently acquired by Qiagen, Germany. Using standard methodology the person skilled in the art can construct an expression vector, express the gene product and isolate the protein essentially as described in WO89/06279 or WO98/20115.

With this information in hand, the expert in the field can also construct and express a gene coding for a protease with an amino acid sequence with 80 % identity to the amino acid sequence of subtilisin 147 by substituting various amino acids. Therefor, he uses standard methodology as described in Sambrook et al. (1989), Molecular Cloning, Cold Spring Harbor University Press, New York, NY, USA or methodology as described in WO89/06279 or WO98/20115. The tests for the proteolytical activity are described in these two international applications or in this invention.

In a further embodiment, a method according to the invention is disclosed characterized in that the amino acid sequence of protease is the amino acid sequence SEQ ID NO 1, a proteolytical derivative thereof having protease activity or the amino acid sequence SEQ ID NO 2. In still another embodiment of the invention, a method according to the invention is disclosed characterized in that the amino acid sequence of the protease according to the invention is encoded by the nucleic acid sequence SEQ ID NO 3, a part thereof coding for an active protease according to the invention or a degenerated version of the nucleic acid sequence SEQ ID NO 3.

In an embodiment of the invention the biological sample is intended to comprise viruses or bacterial cells, as well as isolated cells from multicellular organisms as e.g. human and animal cells such as leucocytes, and immunologically active low and high molecular chemical compounds such as haptens, antigens, antibodies and nucleic acids, blood plasma, cerebral fluid, sputum, stool, biopsy specimens, bone marrow, oral rinses, blood serum, tissues, urine or mixtures thereof. In a preferred embodiment of the invention the biological sample is a fluid from the human or animal body, preferably the biological sample is blood, blood plasma, blood serum or urine. The blood plasma is preferably EDTA, heparin or citrate blood plasma. In an embodiment of the invention the biological sample comprises bacterial cells, eukaryotic cells, viruses or mixtures thereof.

The biological sample can also be of a type used for environmental analysis, food analysis or molecular biology research, e.g. from bacterial cultures, phage lysates. In certain cases the sample can be used without pretreatment in the method according to the invention. In many cases, however, the sample should be lysed using an appropriate method, releasing the biological substances contained in the sample thereby creating a mixture of proteinaceous and non-proteinaceous components derived from the biological sample. Procedures for lysing samples are known by the expert and can be chemical, enzymatic or physical in nature. A combination of these procedures is applicable as well. For instance, lysis can be performed using ultrasound, high pressure, by shear forces, using alkali, detergents or chaotropic saline solutions, or by means of proteases or lipases. With regard for the lysis procedure to obtain nucleic acids, special reference is made to Sambrook et al.: Molecular Cloning, A Laboratory Manual, 2nd Addition, Cold Spring Harbour Laboratory Press, Cold Spring Harbour, NY, USA, and Ausubel et al.: Current Protocols in Molecular Biology 1987, J. Wiley and Sons, NY, USA.

Also described is the case wherein the biological sample comprises a (at least one) glycosylated protein which is partially or fully degraded by the protease according to the invention. Therefore, also described is the use of the protease according to the invention for the partial or full degradation of glycosylated proteins, i.e. proteins with covalently attached carbohydrate moieties.

The method according to the invention can also have further steps after the incubation as binding the target non-proteinaceous component to a material with an affinity thereto, optionally washing and optionally releasing the target non-proteinaceous component from the material with an affinity thereto. Afterwards, the target non-proteinaceous component may be determined or detected by standard analytical methods known to the person skilled in the art and described e.g. in Sambrook et al. (1989), Molecular Cloning, Cold Spring Harbor University Press, New York, NY, USA or in "Bioanalytik", Lottspeich and Zorbas (eds.), 1st edition 1998, Spektrum Akademischer Verlag, Heidelberg, Berlin, Germany.

In order to bind the target non-proteinaceous component to a material with an affinity thereto, the mixture of non-proteinaceous and proteinaceous components is brought in contact with the material with an affinity to the target non-proteinaceous component under conditions in which the target non-proteinaceous component binds to the surface of the material. The conditions for this depend on the type of the target non-proteinaceous component involved, but are basically known to the expert in the field. They also depend on the method by which the target non-proteinaceous component is bound to the surface. For example, if modified nucleic acids are the target non-proteinaceous components, the binding can take place via the groups of nucleic acids that represent the modification, e.g., biotin via binding with streptavidin-coated surfaces.

If unmodified nucleic acids are the target non-proteinaceous components, a direct binding of the nucleic acids to a material with a silica surface is preferred because among other reasons the nucleic acids do not have to be modified and even native nucleic acids can be bound. These processes are described in detail by various documents. In Proc. Natl. Acad. USA 76, 615-691 (1979), for instance, a procedure for binding nucleic acids from agarose gels in the presence of sodium iodide to ground flint glass is proposed. The purification of plasmid DNA from bacteria on glass dust in the presence of sodium perchlorate is described in Anal. Biochem. 121, 382-387 (1982). In DE-A 37 34 442, the isolation of single-stranded M13 phage DNA on glass fiber filters by precipitating phage particles using acetic acid and lysis of the phage particles with perchlorate is described. The nucleic acids bound to the glass fiber filters are washed and then eluted with a methanol-containing Tris/EDTA buffer. A similar procedure for purifying DNA from lambda phages is described in Anal. Biochem. 175, 196-201 (1988). The procedure entails the selective binding of nucleic acids to glass surfaces in chaotropic salt solutions and separating the nucleic acids from contaminants such as agarose, proteins or cell residue. To separate the glass particles from the contaminants, the particles may be either centrifuged or fluids are drawn through glass fiber filters. This is a limiting step, however, that prevents the procedure from being used to process large quantities of samples. The use of magnetic particles to immobilize nucleic acids after precipitation by adding salt and ethanol is more advantageous and described e.g. in Anal. Biochem. 201, 166-169 (1992) and PCT GB 91/00212. In this procedure, the nucleic acids are agglutinated along with the magnetic particles. The agglutinate is separated from the original solvent by applying a magnetic field and performing a wash step. After one wash step, the nucleic acids are dissolved in a Tris buffer. This procedure has a disadvantage, however, in that the precipitation is not selective for nucleic acids. Rather, a variety of solid and dissolved substances are agglutinated as well. As a result, this procedure can not be used to remove significant quantities of any inhibitors of specific enzymatic reactions that may be present. Magnetic, porous glass is also available on the market that contains magnetic particles in a porous, particular glass matrix and is covered with a layer containing streptavidin. This product can be used to isolate biological materials, e.g., proteins or nucleic acids, if they are modified in a complex preparation step so that they bind covalently to biotin. Magnetizable particular adsorbents proved to be very efficient and suitable for automatic sample preparation. Ferrimagnetic and ferromagnetic as well as superparamagnetic pigments are used for this purpose. The most preferred MGPs are those described in WO01/37291.

In detail, the procedure for binding the target nucleic acid to glass particles can be described as follows. It is preferably performed in the presence of chaotropic salts with a concentration of between 1 and 8 mol/l, and preferably between 2 and 6 mol/l. Chaotropic salts can be sodium iodide, sodium perchlorate, guanidinium thiocyanate, guanidinium isothiocyanate or guanidinium hydrochloride. Other substances are also possible. The purification effect results from the behavior of DNA or RNA to bind to material with a glass surface under these conditions i.e. in the presence of certain concentration of a chaotropic agent, higher concentrations of organic solvents or under acidic conditions. To bring the sample in contact with the material with an affinity to the target non-proteinaceous component, the sample is mixed with the material and incubated for a period of time sufficient for the binding to occur. Experts are usually familiar with the duration of the incubation step from procedures for performing treatment with non-magnetic particles. This step can be optimized by determining the quantity of immobilized biological material on the surface at different points in time. Incubation times of between 10 seconds and 30 minutes can be appropriate for nucleic acids. After incubation, the bound target non-proteinaceous component is separated from the liquid. This may be achieved in general by gravity or in the convenient case of nucleic acids bound to magnetic glass particles by separating the material bound to the magnetic particles by applying a magnetic field. For instance, the magnetic particles can be pulled to the wall of the vessel in which incubation was performed. The liquid containing the sample contents that were not bound to the magnetic particles can then be removed. The removal procedure used depends on the type of vessel in which incubation was performed. Suitable steps include removing the liquid via pipetting or aspiration. The material with the bound DNA or RNA may then be washed at least once, preferably with a mixture of 70 volume parts ethanol with 30 volume parts water ("70 % Ethanol"). A wash solution is used that does not cause the (at least one) target non-proteinaceous component to be released from the material surface but that washes away the undesired contaminants as thoroughly as possible. This wash step preferably takes place by incubating the material with the bound target non-proteinaceous component with the wash solution. The material is preferably resuspended during this step. The contaminated wash solution is preferably removed just as in the step described above for binding the biological material. After the last wash step, the material can be dried briefly in a vacuum, or the fluid can be allowed to evaporate. A pretreatment step using acetone may also be performed. Afterwards, the conditions may be reversed, e.g. the concentration of the chaotropic agent or organic solvent is decreased to elute the DNA or RNA bound to the material. Preferably, the process of separating the magnetic glass particles from the rest of the sample is done by pelleting the immobilized biological material, e.g. by gravity force or by the use of a magnet in the case of magnetic glass particles and removal of the supernatant. Then the magnetic glass particles with the immobilized biological material are resuspended in a solution with no or only a low amount of chaotropic agent and/ or organic solvent. Alternatively, the suspension can be diluted with a solution with no or only a low amount of chaotropic agent and/ or organic solvent. Buffers of this nature are known from DE 3724442 and Analytical Biochemistry 175, 196-201 (1988). The elution buffers with a low salt content are in particular buffers with a content of less than 0.2 mol/l. In an especially preferred embodiment, the elution buffer contains the substance Tris for buffering purposes. In another special embodiment, the elution buffer is demineralized water. The solution containing purified DNA or RNA can now be used for other reactions.

For washing and binding steps, preferably liquids are used which are suitable for processes in molecular biology, in particular desoxyribonucleic acid (DNA) or ribonucleic acid (RNA) purification processes which make use of the binding of these substances to glass particles under certain conditions. Preferred liquids comprise alcohols and/ or ketones or any mixtures thereof with water. Alcohols shall include according to the invention preferably primary, secondary or tertiary alcohols of the general formula R-OH where the R stands for the general formula $-(CH_2)_n-CH_3$ with n >= 0. However, other alcohols can also be used if they are suitable for molecular biology purposes as e.g. glycerol. Particularly suitable are the alcohols isopropanol, ethanol or mixtures thereof with water, preferably a mixture of 80 volume parts of isopropanol with 20 volume parts of water. In another embodiment of the invention the liquid comprises ketones as e.g. acetone.

The magnetic glass particles used in the present invention may be provided in different formulations. It is possible to provide them in the form of a tablet, as a powder or preferably as a suspension. These suspensions can contain between 5 to 60 mg/ ml magnetic glass particles (MGPs). In another embodiment of the invention the silica-containing material is suspended in aqueous buffered solutions which may optionally contain a chaotropic agent in a concentration of between 2 and 8 mol/l, and preferably between 4 and 6 mol/l. Chaotropic salts are sodium iodide, sodium perchlorate, guanidinium thiocyanate, guanidinium isothiocyanate or guanidinium hydrochloride. Other compounds known to the expert in the field are also possible. A chaotropic agent according to the present invention is any chemical substance which disturbs the ordered structure of liquid water and has the effect that DNA or RNA binds to the magnetic glass particles if this agent is present in the DNA or RNA containing solution. It is obvious for the artisan to produce suitable aqueous buffered solutions. Buffer systems which suitable for molecular biology purposes may be found e.g. in Sambrook et al. (1989), Molecular Cloning, Cold Spring Harbor University Press, New York, NY, USA. Preferred buffer substances are Tris-(hydroxymethyl)-aminomethane (TRIS), phosphate, N-(2-Hydroxyethyl)piperazine-N'-(2-ethanesulfonic acid) (HEPES), salts thereof or other suitable substances. Additionally, substances may be present which modify the ionic strength of the solution as e.g. NaCl, KCl or $CaCl_2$ or which are metal cation complexing agents as e.g. ethylene-diamine-tetra-acetic acid (EDTA) or the salts thereof. Other biological substances known to the expert in the field may also be present. The method according to the present invention is suitable for the purification of nucleic acids, i.e. RNA or DNA, from complex mixtures with other biological substances containing them. Thereby also mixtures of different nucleic acids may be purified, even mixtures containing a nucleic acid of interest in low abundance. In one embodiment of the invention mixtures of specific nucleic acids are purified, in which the target nucleic acid(s) may be a minor component in terms of concentration (or may be present in low abundance).

The procedure described can be used to isolate native or modified biological material. Native biological material is understood to be material, the structure of which was not irreversibly changed compared with the naturally-occurring biological materials. This does not mean that other components of the sample can not be modified, however. Modified biological materials include materials that do not occur in nature, e.g., nucleic acids that are modified by attaching to them groups that are reactive, detectable or capable of immobilization. An example of this are biotinylated nucleic acids.

After the steps described above, the non-proteinaceous components isolated using the method according to the invention can now be used further as necessary. For instance, they can be used as a substrate for various enzymatic reactions. When nucleic acids are involved, they can be used for sequencing, radioactive or non-radioactive labelling, amplification of one or more of the sequences they contain, transcription, hybridization with labelled probe nucleic acids, translation or ligation. Therefore, in a more preferred embodiment of the invention the method comprises the step of releasing the bound (at least one) target non-proteinaceous component from the material with an affinity thereto. If desired, the (at least one) target non-proteinaceous component purified in this manner can be separated from the material as described above.

In a preferred embodiment of the invention the method comprises the step of detecting or determining a target non-proteinaceous component. A preferred embodiment of the invention are therefore the above-described purification method followed by a determination or detection step or purification methods followed by an amplification and determination or detection step. In the case of nucleic acids, the target nucleic acid or nucleic acids of interest may be contained in a matrix of non-target nucleic acids, and may even be a minor component in said mixture of specific nucleic acids. Suitable DNA detection methods are known to the expert in the field and are described in standard textbooks as Sambrook et al.: Molecular Cloning, A Laboratory Manual, 2nd Addition, Cold Spring Harbour Laboratory Press, Cold Spring Harbour, NY and Ausubel et al.: Current Protocols in Molecular Biology 1987, J. Wiley and Sons, NY. There may be also further purification steps before the DNA detection step is carried out as e.g. a precipitation step. The detection methods may include but are not limited to the binding or intercalating of specific dyes as ethidiumbromide which intercalates into the doublestranded DNA and changes its fluorescence thereafter. The purified DNA may also be separated by electrophoretic methods optionally after a restriction digest and visualized thereafter. There are also probe-based assays which exploit the oligonucleotide hybridisation to specific sequences and subsequent detection of the hybrid. It is also possible to sequence the DNA after further steps known to the expert in the field. Other methods apply a diversity of DNA sequences to a silicon chip to which specific probes are bound and yield a signal when a complementary sequences bind.

In a preferred embodiment of the invention the mixture of non-proteinaceous and proteinaceous components comprises nucleic acids whereby the nucleic acids comprise DNA or RNA or both.

A preferred embodiment of the invention is related to a method for the analysis of a target nucleic acid component of a mixture non-proteinaceous components, which comprise nucleic acids, and proteinaceous material derived from a biological sample comprising the steps of

a) incubating the mixture with a protease having an amino acid sequence which is at least 80 % identical to the amino acid sequence SEQ ID NO: 1

b) optionally amplifying the target nucleic acid component, and

c) determining or detecting the target nucleic acid component.

In a preferred embodiment of the invention, the amount of the target nucleic acid component is determined.

In a preferred embodiment of the invention the amino acid sequence of protease is the amino acid sequence SEQ ID NO 1, a proteolytical derivative thereof having protease activity or the amino acid sequence SEQ ID NO 2. In yet another preferred embodiment of the invention the amino acid sequence of the protease according to the invention is encoded by the nucleic acid sequence SEQ ID NO 3, a part thereof or a degenerated version of the nucleic acid sequence SEQ ID NO 3. In still another embodiment of the invention the biological sample is intended to comprise viruses or bacterial cells, as well as isolated cells from multicellular organisms as e.g. human and animal cells such as leucocytes, and immunologically active low and high molecular chemical compounds such as haptens, antigens, antibodies and nucleic acids, blood plasma, cerebral fluid, sputum, stool, biopsy specimens, bone marrow, oral rinses, blood serum, tissues, urine or mixtures thereof. In a preferred embodiment of the invention the biological sample is a fluid from the human or animal body, preferably the biological sample is blood, blood plasma, blood serum or urine. The blood plasma is preferably EDTA, heparin or citrate blood plasma. In an embodiment of the invention the biological sample comprises bacterial cells, eukaryotic cells, viruses or mixtures thereof.

In a preferred embodiment of the invention the mixture of nucleic acids and proteinaceous material comprises desoxyribonucleic acid (DNA) or ribonucleic acid (RNA) or both, preferably the DNA or RNA or both is derived from a (at least one) virus or a (at least one) microorganism. The virus can be hepatitis A virus (HAV), hepatitis B virus (HBV), hepatitis C virus (HCV), the human immunodeficiency virus (HIV), the human papilloma virus (HPV) or parvovirus B19.

In a preferred embodiment of the invention a (at least one) target nucleic acid component and the other nucleic acids are purified essentially as described above. Then the (at least one) target nucleic acid component is further manipulated and detected, i.e. it is amplified with the polymerase chain reaction which specifically amplifies target sequences to detectable amounts. Other possible amplification reactions are the ligase Chain Reaction (LCR, Wu and Wallace, 1989, Genomics 4:560-569 and Barany, 1991, Proc. Natl. Acad. Sci. USA 88:189-193); Polymerase Ligase Chain Reaction (Barany, 1991, PCR Methods and Applic. 1:5-16); Gap-LCR (PCT Patent Publication No. WO 90/01069); Repair Chain Reaction (European Patent Publication No. 439,182 A2), 3SR (Kwoh et al., 1989, Proc. Natl. Acad. Sci. USA 86:1173-1177; Guatelli et al., 1990, Proc. Natl. Acad. Sci. USA 87:1874-1878; PCT Patent Publication No. WO 92/0880A), and NASBA (U.S. Pat. No. 5,130,238). Further, there are strand displacement amplification (SDA), transciption mediated amplification (TMA), and Qβ-amplification (for a review see e.g. Whelen and Persing (1996). Annu. Rev. Microbiol. 50, 349-373; Abramson and Myers, 1993, Current Opinion in Biotechnology 4:41-47).

A particularly preferred detection method is the TaqMan® method disclosed in WO92/02638 and the corresponding US patents US 5,210,015, US 5,804,375, US 5,487,972. This method exploits the exonuclease activity of a polymerase to generate a signal. In detail, the target nucleic acid component is detected by a process comprising contacting the sample with an oligonucleotide containing a sequence complementary to a region of the target nucleic acid component and a labeled oligonucleotide containing a sequence complementary to a second region of the same target nucleic acid component sequence strand, but not including the nucleic acid sequence defined by the first oligonucleotide, to create a mixture of duplexes during hybridization conditions, wherein the duplexes comprise the target nucleic acid annealed to the first oligonucleotide and to the labeled oligonucleotide such that the 3'-end of the first oligonucleotide is adjacent to the 5'-end of the labeled oligonucleotide. Then this mixture is treated with a template-dependent nucleic acid polymerase having a 5' to 3' nuclease activity under conditions sufficient to permit the 5' to 3' nuclease activity of the polymerase to cleave the annealed, labeled oligonucleotide and release labeled fragments. The signal generated by the hydrolysis of the labeled oligonucleotide is detected and/ or measured. TaqMan® technology eliminates the need for a solid phase bound reaction complex to be formed and made detectable. In more general terms, a procedure for the purification of a target nucleic acid component followed by a detection step is disclosed wherein the amplification and/ or detection reaction is a homogeneous solution-phase.

In another preferred embodiment of the invention the nucleic acids including the target nucleic acid component are bound to a material with an affinity thereto before they are optionally amplified or determined or detected. After binding they are optionally washed and optionally released from the material with an affinity thereto essentially as described above. Therefore, a preferred embodiment of the invention is related to a method for the analysis of a target nucleic acid component of a mixture non-proteinaceous components, which comprise nucleic acids, and proteinaceous material derived from a biological sample comprising the steps of

a) incubating the mixture with a protease according to the invention

b) binding the target non-proteinaceous component to a material with an affinity thereto,

c) optionally washing and optionally releasing the target nucleic acid component from the material with an affinity thereto,

d) optionally amplifying the target nucleic acid component, and

e) determining or detecting the target nucleic acid component.

In the most preferred embodiment, the steps c) and d) are not optional, i.e. that the bound target nucleic acid component is washed and released from the material with an affinity thereto and the target nucleic acid component is amplified before it is determined or detected. Preferably the amount of the target nucleic acid component is determined.

The material with an affinity to nucleic acids and the target nucleic acid component comprises a material with a silica surface, preferably the material with a silica surface is a glass, most preferably the material with an affinity to nucleic acids is a composition comprising magnetic glass particles. The steps are performed essentially as already describe above. In summary, magnetic glass particles are added to the lysis mixture comprising the nucleic acids including the target nucleic acid component.

After a suitable period of time for adsorption to take place - which can be optimized by mechanical agitation - the particles are separated from the surrounding fluid that contains additional components that are not to be detected. This is performed preferably by applying a magnetic field by placing a magnet against the vessel wall and removing the remaining liquid from the tube. To remove further contaminants that may still be present, a wash step is preferably performed with a fluid that does not cause the nucleic acids and the (at least one) target nucleic acid component to be released from the glass surface. An elution buffer having reagent conditions under which the nucleic acids and the target nucleic acid component are not bound to the glass surface and are eluted is added to remove the nucleic acids including the target nucleic acid component from the glass surface. These conditions are low salt conditions in particular. Depending on the intended further use of the nucleic acids and the target nucleic acid component, the fluid can now be separated from the particles and processed further. This separation step is preferably performed via application of a magnetic field so that the particles are separated from the eluate. The most preferred magnetic glass particles for this method are described in WOO 1/37291.

Preferably the method according to the invention is used for diagnostic analysis or bioanalytics.

In a preferred embodiment of the invention the protease according to the invention is used in research, bioanalytics or diagnostics. In further preferred embodiments the protease according to the invention is used for the analysis of a target non-proteinaceous component of a mixture of non-proteinaceous and proteinaceous components derived from a biological sample, for the enrichment of a target non-proteinaceous component of a mixture of non-proteinaceous and proteinaceous components derived from a biological sample or for the purification or isolation of a target non-proteinaceous component of a mixture of non-proteinaceous and proteinaceous components derived from a biological sample. Preferably the target non-proteinaceous component is a nucleic acid, preferably from a virus or a microorganism, or the mixture of non-proteinaceous and proteinaceous components comprises nucleic acids. Preferred viruses are hepatitis B virus, hepatitis C virus or the human immunodeficiency virus or the other viruses described above.

The invention further contemplates a kit of parts suitable to be used in diagnostics and/or for the purification of nucleic acids characterized in that it contains a protease having an amino acid sequence, which is at least 80 % identical to the amino acid sequence SEQ ID NO:1. In a preferred embodiment of the invention the amino acid sequence of protease is the amino acid sequence SEQ ID NO 1, a proteolytical derivative thereof having protease activity or the amino acid sequence SEQ ID NO 2, preferably the amino acid sequence of the protease according to the invention is encoded by the nucleic acid sequence SEQ ID NO 3, a part thereof coding for an active protease or a degenerated version of the nucleic acid sequence SEQ ID NO 3. Such kits known in the art further comprise plastics ware which can be used during the sample preparation procedure as e.g. microtitre plates in the 96 or 384 well format or just ordinary reaction tubes manufactured e.g. by Eppendorf, Hamburg, Germany and all other reagents for carrying out the method according to the invention. Therefore, the kit can additionally contain a material with an affinity to nucleic acids (and the target nucleic acid component), preferably the material with an affinity to nucleic acids target nucleic acid component) comprises a material with a silica surface. Preferably, the material with a silica surface is a glass. Most preferably, the material with an affinity to nucleic acids is a composition comprising magnetic glass particles. The kit can further or additionally comprise a lysis buffer containing e.g. chaotropic agents, detergents or alcohols or mixtures thereof which allows the lysis of cells. These components of the kit according to the invention may be provided separately in tubes or storage containers. Depending on the nature of the components, these may be even provided in a single tube or storage container. The kit may further or additionally comprise a washing solution which is suitable for the washing step of the magnetic glass particles when DNA or RNA is bound thereto. This washing solution may contain ethanol and/ or chaotropic agents in a buffered solution or solutions with an acidic pH without ethanol and/ or chaotropic agents as described above. Often the washing solution or other solutions are provided as stock solutions which have to be diluted before use. The kit may further or additionally comprise an eluent or elution buffer, i.e. a solution or a buffer (e.g. 10 mM Tris, 1 mM EDTA, pH 8.0) or pure water to elute the DNA or RNA bound to the magnetic glass particles. Further, additional reagents or buffered solutions may be present which can be used for the purification process of a nucleic acid, i.e. DNA or RNA.

A preferred embodiment of the present invention is to use the method or the kit of the present invention in automatable methods as e.g. described in WO 99/ 16781. Automatable method means that the steps of the method are suitable to be carried out with an apparatus or machine capable of operating with little or no external control or influence by a human being. Automatized method means that the steps of the automatable method are carried out with an apparatus or machine capable of operating with little or no external control or influence by a human being. Only the preparation steps for the method may have to be done by hand, e.g. the storage containers have to filled up and put into place, the choice of the samples has to be done by a human being and further steps known to the expert in the field, e.g. the operation of the controlling computer. The apparatus or machine may e.g. add automatically liquids, mix the samples or carry out incubation steps at specific temperatures. Typically, such a machine or apparatus is a robot controlled by a computer which carries out a program in which the single steps and commands are specified. Preferred automatized methods are those which are carried out in a high-throughput format which means that the methods and the used machine or apparatus are optimized for a high-throughput of samples in a short time. In another embodiment of the invention the methods or the kits according to the present invention are used in semi-automatized process which means that some reaction steps may have to be done manually. In a preferred embodiment of the invention, a suspension containing MGPs according to the present invention is taken from a storage container and partial volumes are added to different reaction vessels. Reaction vessels may be reaction tubes made from plastics eventually in mictrotitreplate format contain 96 or 384 or more wells where a reaction can be carried out. However, these vessels may be made from other material e.g. from steel.

In preferred embodiments of the invention the kit according to the invention is used for the purification of nucleic acids in research, bioanalytics or diagnostics. The kit according to the invention or the method according to the invention can be used in a high-throughput format, i.e. in an automatized method which allows the analysis of a high number of different samples in a very short time.

The person skilled in the art knows from the teachings and the example of the present invention how to identify other proteases performing in an equivalent manner as the protease according to the invention, i.e. the protease esperase. Thereby, it is also possible to identify variant or mutant proteins of esperase performing in an equivalent manner to esperase. "Mutant amino acid sequence," "mutant protein" or "mutant polypeptide" refers to a polypeptide having an amino acid sequence which varies from a native sequence or is encoded by a nucleotide sequence intentionally made variant from a native sequence. "Mutant protein," "variant protein" or "mutein" means a protein comprising a mutant amino acid sequence and includes polypeptides which differ from the amino acid sequence of native esperase due to amino acid deletions, substitutions, or both. "Native sequence" refers to an amino acid or nucleic acid sequence which is identical to a wild-type or native form of a gene or protein.

To find these variant or mutant proteins, he will prepare solutions identical to the reagents and buffers described in Example 1 whereby esperase is used as a standard for the determination of the protease activity. Primarily, the expert in the field will analyze the protease of interest as described in the Chromatographic Analysis of Plasma Protein Digestion Protocol (see Example 3). The protease in question will further be analyzed by its properties in sample preparation with subsequent PCR amplification and detection of the amplified product (see Example 2). Of further interest for comparison with the disclosed enzyme esperase is the investigation of the storage stability (see Example 6) or the evaluation of the enzymatic activity in the presence of chaotropic agents (see Example 5). Taking the results of these investigations into account, the expert in the field can decide whether a protease of interest performs in an equivalent manner as the protease esperase disclosed by the present invention.

A further embodiment of the invention is an aequeous composition of a protease according to the invention, i.e. a protease which is at least 80 % identical to the amino acid sequence SEQ ID NO:1 whereby the composition comprises 10 mM Tris acetate, 5 mM calcium chloride, 5 mM calcium acetate, 1 mM EDTA, 50 % (V/V = Volume/Volume) glycerin with a pH value of 5.5. This composition is an ideal storage buffer for esperase (see example 6), The expert skilled in the art is able to modify the composition of the buffer taking the teachings of example 5 into account as long as the protease according to the invention is equally stable in the modified buffer composition. In a further embodiment, the amino acid sequence of the protease in the above-desribed composition is the amino acid sequence SEQ ID NO 1, a proteolytical derivative thereof having protease activity or the amino acid sequence SEQ ID NO 2. In another embodiment, the amino acid sequence of the protease according to the invention in the above-described composition is encoded by the nucleic acid sequence SEQ ID NO 3, a part thereof or a degenerated version of the nucleic acid sequence SEQ ID NO 3. The composition according to the invention can be used in sample preparation or sample preparation methods, in particular in the methods according to the invention, for the purification of nucleic acids or in diagnostics or diagnostical analysis. The following examples, references, sequence listing and figures are provided to aid the understanding of the present invention, the true scope of which is set forth in the appended claims.

## Example 1: Reagents and Buffers

### 1.1 Proteases

The following proteases have been tested for their suitability in the sample preparation process:

| | |
|---|---|
| Alcalase | (Novo Nordisk) |
| Proteinase K (60 mg/ml) | Roche Diagnostics, Cat. No.1 964 372 |
| Subtilisin A (19 mg/ml) | (Novo Nordisk) |
| Esperase (24 mg/ml) | (Novo Nordisk) |
| Chirazym (31 mg/ml) | |
| Novozyme 539 | (Novo Nordisk) |
| Novo 47002 | (Novo Nordisk) |
| Novocor PL | (Novo Nordisk) |
| Pronase | (Roche Diagnostics, Cat. No. 165 921) |

### 1.2 Buffers:

#### 1.2.1 Lysis- and Binding Buffer:

Lysis-and binding buffer has been prepared from:

5 M Guanidiumthiocyanate

15% Polydocanol

1% Dithiothreitol (DTT)

15 mM Bis-TRIS, pH 6.0

#### 1.2.2 Washing buffer:

Washing buffer had the following composition:

50 % Ethanol

50 mM NaCl

10 mM Bis-TRIS, pH 6.0

#### 1.2.3 Elution Buffer:

Elution buffer has been RNase-free destilled water.

**1.3 Magnetic Glass Particles:**

Magnetic glass particles as described in WO01/37291 have been suspended in isopropanol at a concentration of 6 mg/ml. The said magnetic glass particles can also be taken from the MagNA Pure LC DNA Isolation Kit I (Roche, Mannheim, Germany).

**1.4 Buffers for the Protease Activity Assay**

| | |
|---|---|
| Buffer: | 50 mM Tris/ HCl pH 8.2 |
| | 10 mM calcium chloride |
| Substrate solution: | 200 mM Suc-Ala-Ala-Pro-Phe-p-nitroanilide in dimethylsulfoxide (DMSO) |

**Example 2 Sample Preparation Method and Polymerase Chain Reaction**

**2.1 Protease digestion and lysis:**

80 µl protease solution is mixed with 420 µl sample material (e.g. plasma with a specific virus concentration) and mixed. 500 µl lysis- and binding buffer are added and the solution is mixed for 10 minutes at room temperature.

**2.2 Binding:**

500 µl of the suspension of magnetic glass particles in isopropanol are added and the solution is mixed for 20 minutes at room temperature.

**2.3 Washing:**

After the binding step the magnetic glass particles are separated from the solution by a magnet and washed five times with 750 µl washing buffer per wash cycle.

**2.4 Elution:**

After the last wash cycle the magnetic glass particles are separated by a magnet from the suspension and the washing buffer is sucked off from the magnetic glass particles and 100µ l elution buffer are added. The suspension is mixed and incubated for 15 minutes at 80°C. After the elution step the magnetic glass particles are separated again by a magnet and the supernatant containing the viral nucleic acid is harvested.

**2.5 Protocol Amplification/Detection:**

With the exception of the primers all reagents were purchased from Roche Molecular Biochemicals.

**Master Mix HCV:**

| Reagent | conc./PCR |
|---|---|
| Bicine Buffer (pH 8.3) | 1 x |
| MnOAc | 2.5 mM |
| dNTP Mix with dUTP | |
| dUTP | 0.6 mM |
| dATP/dCTP/dGTP | 0.2 mM each |

(continued)

| Reagent | conc./PCR |
|---|---|
| Primer KY 80 (F) | 300 nMol |
| Primer KY 78-bio (R) | 300 nMol |
| Tth-Polymerase | 10 U |
| Uracil-N-glycosylase (UNG) | 2 U |

**Master Mix HIV:**

| Reagent | conc./PCR |
|---|---|
| Bicine Buffer (pH 8.3) | 1 x |
| MnOAc | 1.25 mM |
| dNTP Mix with dUTP | |
| dUTP | 0.6 mM |
| dATP/dCTP/dGTP | 0.2 mM each |
| Primer SK 462-bio (F) | 200 nMol |
| Primer SK 431-bio (R) | 200 nMol |
| Tth-Polymerase | 15 U |
| UNG | 2 U |

**Master Mix HBV:**

| Reagent | conc./PCR |
|---|---|
| DNA-Master Mix | 1x |
| $MgCl_2$ | 3.0 mM |
| Primer 1 (F) | 200 nMol |
| Primer 2(bio (R)) | 200 nMol |
| UNG | 2 U |

20 µl of the eluate from the sample preparation process which contains the target nucleic acid, e.g. viral RNA (HCV, HIV) or viral DNA (HBV) are mixed which 100 µl master mix. Amplification is performed on a Perkin-Elmer Thermocycler 9600 with the following thermocycler programms:

**HCV:**

| | | |
|---|---|---|
| UNG step | 1x | 10 min 37°C |
| RT step | 1x | 30 min 60°C |
| | 1x | 1 min 95°C |
| PCR | 2x | 10 sec 95°C |
| | | 20 sec 60°C |
| | 33x | 15 sec 90°C |
| | | 20 sec 60°C |
| | 1x | 7 min 72°C |

**HIV:**

| | | |
|---|---|---|
| UNG step | 1x | 10 min 37°C |
| RT step | 1x | 30 min60°C |
| PCR | 4x | 10 sec 95°C |
| | | 10 sec 55°C |
| | | 10 sec 72°C |
| | 31x | 10 sec 90°C |
| | | 10 sec 60°C |
| | | 10 sec 72°C |

**HBV:**

| | | |
|---|---|---|
| UNG step | 1x | 10 min 37°C |
| PCR | 35x | 30 sec 92°C |
| | | 30 sec 55°C |

(continued)

40 sec 72°C

For the detection of the amplified material, a very sensitive nonisotopic approach based on electrochemiluminescence (ECL) was used. Ruthenium-tris(bipyridyl)-labeled oligonucleotides (capture probes) were hybridized specifically to the biotinylated denatured amplicons. Subsequent, this hybrid was bound to the surface of streptavidin-coated magnetic beads. After the beads were captured on an electrode by using a permanent magnet, the ECL reaction of the ruthenium label was triggered by voltage application. For details of the ECL detection process, see Hoyle et al. (13). The totally automated ECL detection was performed on an instrumental platform (preprototype of Elecsys 1010; Boehringer Mannheim GmbH).

**HCV:**

KY80: SEQ ID NO: 4

KY78: SEQ ID NO: 5

Probe: SEQ ID NO: 6

**HIV:**

SK 462: SEQ ID NO: 7

SK 431: SEQ ID NO: 8

Probe: SEQ ID NO: 9

**HBV:**

Primer 1: SEQ ID NO: 10

Primer 2: SEQ ID NO: 11

Probe: SEQ ID NO: 12

**Result**

| Virus | Proteinase K (ECL counts x $10^{-3}$) | Pronase (ECL counts x $10^{-3}$) | Subtilisin A (ECL counts x $10^{-3}$) | Esperase (ECL counts x $10^{-3}$) | Chirazym (ECL counts x $10^{-3}$) |
|---|---|---|---|---|---|
| HIV | 278 | 62 | 62 | 210 | 214 |
| HCV | 184 | 22 | 49 | 179 | 249 |
| HBV | 371 | 30 | 241 | 300 | 446 |

Only the use of esperase and chirazym for the degradation of plasma proteins in the sample preparation process results in an ECL signal comparable to the signal generated by the use of proteinase K in the sample preparation process.

**Example 3 Protocol Chromatographic Analysis of Plasma Protein Digestion:**

Protein digestion and lysis were carried out as described. Each 100 µl of the lysated solution were injected onto an high pressure liquid chromatography instrument (HPLC) (Dionex, Gynkothek) and separated on an reversed phase column (C4, Vydac, 4.6 mm x 150 mm) in a linear gradient of 0 - 80 % acetonitrile in 0.1 % trifluoroacetic acid (TFA). Peaks were detected at a wavelength of 220 nm and 280 nm.

| **Protease** | **Plasma Protein Digestion** | |
|---|---|---|
| | **With unstressed Protease** | **with Protease stressed by thermal treatment (after 3 day incubation at 45 °C in storage buffer*** |
| Esperase | ++ | ++ |
| Proteinase K | ++ | ++ |
| Pronase | ++ | - |
| Subtilisin A | ++/+ | + |
| Alcalase | + | not tested |
| Novozyme 539 | + | not tested |
| Novo 47002 | - | not tested |
| Novocor PL | - | not tested |
| * Storage buffer composition: 10 mM Tris acetate, 5 mM calcium chloride, 5 mM calcium acetate, 1 mM EDTA, 50 % (V/V) glycerin with a pH value of 5.5 | | |

In Figure 1, the comparison of the digestion of EDTA plasma versus citrate plasma with Esperase (see Figure 1a) and proteinase K (see Figure 1b) is shown.

**Example 4 Evaluation of the pH optimum**

The pH optimum of esperase was compared to the pH optimum of proteinase K using the buffers as basically described under 1.1.4 with a varying pH. The pH optimum was more in the neutral pH region as compared to proteinase K (see Figure 2).

Sample: 10 mg protein are dissolved in 1 ml dest. water. Before the determination, the sample is diluted with dest. water so that the increase in the extinction in the test is between 0.02 and 0.05 E.

Sample buffer:

- pH-range: 5.5 bis 7.5: 50 mM Bis-Tris + 10 mM $CaCl_2$ are adjusted with 2 N HCl or 2 N NaOH to the respective pH.
- pH-range 7.5 bis 9.5: 50 mM Tris-Base + 10 mM $CaCl_2$ are adjusted with 2 N HCl or 2 N NaOH to the respective pH.

Substrate: Suc-Ala-Ala-Pro-Phe-p-nitroanilide (200 mM dissolved in Dimethyl sulfoxide (DMSO)).

Measurement:

- Pipetting scheme: 2.00 ml sample buffer
  0.02 ml substrate
  0.05 ml sample

- Temperature for measurement: 25°C
- Wavelength for measurement: 405 nm
- Evaluation: The linear increase in extinction (de/min) is determined between 2 and 6 min.
- Layer thickness : 1 cm

$$\text{Activity} = \frac{2.07 * dE/min}{10.4\ (\varepsilon) * 0.05 * 1} * \text{dilution (U/ml)}$$

Relative Activity: For each sample, the highest measured activity is regarded as the value of 100 % and the activities at other pH-values are evaluated by determining the percental relation to this value.

**Example 5 Evaluation of the enzymatic activity in the presence of chaotropic agents**

The enzymatic activity of esperase was compared to the enzymatic activity of proteinase K in the presence of chaotropic agents using the buffers as basically described under 1.1.4 with increasing amounts of chaotropic agent. Esperase retained more activity in the presence of chaotropic agents (see Figure 3 and Figure 4). This lower residual activity is advantageous as the protein digestion by esperase is very quick in the presence of chaotropic agent (≤ 1 min) and as esperase has a low residual activity. This is of advantage as less active esperase is transferred into the amplification reaction where it may disturb the amplification reaction.

- Protease solution: 20 mg/ml Protease
- Sample: 500 μl chaotropic agent
  50 μl protease solution.
- The activity of the protease is determined in various solutions. Then, the sample is incubated for 15 min at 25°C and the residual activity determined in various agents.

Determination of the activity:

- Test buffer: 50 mM Tris.HCl pH = 8.2; 10 mM $CaCl_2$
- Substrate: 200 mM Suc-Ala-Ala-Prp-Phe-p-nitroanilide in DMSO
- Measuring temperature: 25°C
- Measuring wavelength: 405 nm

Evaluation: see evaluation of the pH Optimum.

**Example 6 - Storage Stability:**

The stability of the proteases was determined by following the proteolytic activity under thermal stress in storage buffer (composition: 10 mM Tris acetate, 5 mM calcium chloride, 5 mM calcium acetate, 1 mM EDTA, 50 % (V/V) Glycerin with a pH value of 5.5). A kinetic assay with Suc-Ala-Ala-Pro-Phe-p-nitroanilide as a substrate was used. Shortly before use the protease sample has to be diluted to a concentration of 1-3 μg/ml with distilled water. 2 ml buffer were mixed with 0.02 ml substrate and 0.05 ml diluted sample. The release of p-nitroaniline from the substrate at 25°C was measured photometrically at 405 nm. The time-curve of the stability of Esperase in comparison to proteinase K is shown in Figure 5. The result of this experiment is that it could be shown that Esperase is very stable in storage buffer even after a prolonged period of time.

| Protease | Remaining activity after 3 day incubation at 45 °C in storage buffer (composition see above) |
|---|---|
| Esperase | 88 % |
| Proteinase K | 94 % |
| Pronase | 89 % |
| Subtilisin A | 45 % |

**List of References**

"Bioanalytik", Lottspeich and Zorbas (eds.), 1st edition 1998, Spektrum Akademischer Verlag, Heidelberg, Berlin, Germany
Abramson and Myers, 1993, Current Opinion in Biotechnology 4:41-47
Anal. Biochem. 121, 382-387 (1982)
Anal. Biochem. 175, 196-201 (1988)
Anal. Biochem. 201, 166-169 (1992)
Ausubel et al.: Current Protocols in Molecular Biology 1987, J. Wiley and Sons, NY, USA
Barany, 1991, PCR Methods and Applic. 1:5-16
Barany, 1991, Proc. Natl. Acad. Sci. USA 88:189-193
DE 3724442
EP 110165
EP 396 608
EP 439 182
GB 91/00212
Guatelli et al., 1990, Proc. Natl. Acad. Sci. USA 87:1874-1878
Hoyle, N. R., B. Eckert, and S. Kraiss. 1996
Jacobs et al., 1985, Nucl. Acids Res. 13: 8913-8926
Jany and Mayer, 1985, Biol. Chem. Hoppe-Seyler 366: 485-492
Jany and Mayer, 1985, Biol. Chem. Hoppe-Seyler 366: 584-492
Kraut, 1977, Ann. Rev. Biochem. 46: 331-358
Kurihara et al., 1972, J. Biol. Chem. 247: 5629-5631
Kwoh et al., 1989, Proc. Natl. Acad. Sci. USA 86:1173-1177
Meloun et al., 1985, FEBS Lett. 183: 195-200
Nedkov et al., 1985, Biol. Chem. Hoppe-Seyler 366: 421-430
Priest, 1977, Bacteriological Rev. 41: 711-753
Proc. Natl. Acad. USA 76, 615-691 (1979)
Sambrook et al.: Molecular Cloning, A Laboratory Manual, 2nd Addition, Cold Spring Harbour Laboratory Press, Cold Spring Harbour, NY, USA
Stahl and Ferrari, 1984, J. Bacteriol. 158: 411-418
Svendsen et al., 1986, FEBS Lett. 196: 228-232
US 3,723,250
US 4,683,195
US 5,130,238
US 5,210,015
US 5,386,024
US 5,487,972
US 5,741,694
US 5,804,375
Vasantha et al., 1984, J. Bacteriol. 159: 811-819

Wallace, 1989, Genomics 4:560-569
Walsh, 1979, Enzymatic Reaction Mechanisms. W. H. Freeman and Company, SanFrancisco, Chapter 3
Whelen and Persing (1996). Annu. Rev. Microbiol. 50, 349-373
White, Handler, and Smith, 1973 "Principles of Biochemistry," Fifth Edition, McGraw-Hill Book Company, N.Y., pp. 271-272
WO 89/06279
WO 90/01069
WO 92/02638
WO 92/0880A
WO 98/04730
WO 98/20115

## Claims

**1.** A method for the analysis of a target non-proteinaceous component of a mixture of non-proteinaceous and proteinaceous components derived from a biological sample comprising the step of incubating the mixture with a protease having an amino acid sequence which is at least 80% identical to the amino acid sequence SEQ ID NO 1, a proteolytical derivative thereof having protease activity or the amino acid sequence SEQ ID NO 2.

**2.** A method according to claim 1,
**characterized in that**
the amino acid sequence of the protease is identical to the amino acid sequence SEQ ID NO 1, a proteolytical derivative thereof having protease activity or the amino acid sequence SEQ ID NO 2.

**3.** A method according to any of the claims 1 to 2,
**characterized in that**
the amino acid sequence of the protease according to any of the claims 1 to 2 is encoded by the nucleic acid sequence SEQ ID NO 3, a part thereof or a degenerated version of.the nucleic acid sequence SEQ ID NO 3.

**4.** A method according to any of the claims 1 to 3,
**characterized in that**
the biological sample is a fluid from the human or animal body.

**5.** A method according to any of the claims 1 to 4,
**characterized in that**
the biological sample is blood, blood plasma, blood serum or urine.

**6.** A method according to any of the claims 1 to 5,
**characterized in that**
the biological sample comprises bacterial cells, eukaryotic cells, viruses or mixtures thereof.

**7.** A method according to any of the claims 1 to 6,
**characterized in that**
after the incubation step the target non-proteinaceous component is bound to a material with an affinity thereto, optionally washed and optionally released from the material with an affinity thereto.

**8.** A method according to any of the claims 1 to 7,
**characterized in that**
the mixture of non-proteinaceous and proteinaceous components comprises nucleic acids.

**9.** A method according to claim 8,
**characterized in that**
the nucleic acids comprise DNA or RNA or both.

**10.** A method for the analysis of a target nucleic acid component of a mixture of non-proteinaceous components, which comprise nucleic acids, and proteinaceous components whereby the mixture is derived from a biological sample comprising the steps of

a) incubating the mixture with a protease having an amino acid sequence which is at least 80 % identical to the amino acid sequence SEQ ID NO 1, a proteolytical derivative thereof having protease activity or the amino acid sequence SEQ ID NO 2,
b) optionally amplifying the target nucleic acid component, and
c) determining or detecting the target nucleic acid component.

**11.** A method according to claim 10,
**characterized in that**
the amino acid sequence of the protease is identical to the amino acid sequence SEQ ID NO 1, a proteolytical derivative thereof having protease activity or the amino acid sequence SEQ ID NO 2.

**12.** A method according to any of the claims 10 to 11,
**characterized in that**
the amino acid sequence of the protease is encoded by the nucleic acid sequence SEQ ID NO 3, a part thereof or a degenerated version of the nucleic acid sequence SEQ ID NO 3.

**13.** A method according to any of the claims 10 to 12,
**characterized in that**
the biological sample is a fluid from the human or animal body.

**14.** A method according to any of the claims 10 to 13,
**characterized in that**
the biological sample is blood, blood plasma, blood serum or urine.

**15.** A method according to any of the claims 10 to 14,
**characterized in that**
the nucleic acids comprise DNA or RNA or both.

**16.** A method according to claim 15,
**characterized in that**
the DNA or RNA or both is derived from a virus or a microorganism.

**17.** A method according to claim 16,
**characterized in that**
the virus is hepatitis B virus, hepatitis C virus or the human immunodeficiency virus.

**18.** A method according to any of the claims 10 to 17,
**characterized in that**
the target nucleic acid component is amplified with the polymerase chain reaction.

**19.** A method according to any of the claims 10 to 18,
**characterized in that**
after step a) the nucleic acids and the target nucleic acid component are bound to a material with an affinity to nucleic acids, optionally washed and optionally released from the material with an affinity to nucleic acids.

**20.** A method according to claim 19,

**characterized in that**
the material with an affinity to nucleic acids and the target nucleic acid component comprises a material with a silica surface.

**21.** A method according to claim 20,
**characterized in that**
the material with a silica surface is a glass.

**22.** A method according to any of the claims 19 to 20,
**characterized in that**
the material with an affinity to nucleic acids and the target nucleic acid component is a composition comprising magnetic glass particles.

**23.** Use of the method according to any of the claims 1 to 22 for the diagnostic analysis.

**24.** Use of a protease as specified in any of the claims 10 to 12 in diagnostics.

**25.** Use of a protease as specified in any of the claims 10 to 12 for the analysis of a target non-proteinaceous component of a mixture of non-proteinaceous and proteinaceous components derived from a biological sample.

**26.** Use of a protease as specified in any of the claims 10 to 12 for the enrichment of a target non-proteinaceous component of a mixture of non-proteinaceous and proteinaceous components derived from a biological sample.

**27.** Use of a protease as specified in any of the claims 10 to 12 for the purification or isolation of a target non-proteinaceous component of a mixture of non-proteinaceous and proteinaceous components derived from a biological sample.

**28.** Use according to any of the claims 25 to 27,
**characterized in that**
the target non-proteinaceous component is a nucleic acid.

**29.** Use according to claim 28,
**characterized in that**
the nucleic acid is from a virus or a microorganism.

**30.** A kit of parts,
**characterized in that**
it contains a protease having an amino acid sequence, which is at least 80% identical to the amino acid sequence SEQ ID NO 1, a proteolytical derivative thereof having protease activity or the amino acid sequence SEQ ID NO 2 and a material with an affinity to nucleic acids which comprises a material with a silica surface.

**31.** A kit according to claim 30,
**characterized in that**
the amino acid sequence of the protease is identical to the amino acid sequence SEQ ID NO 1, a proteolytical derivative thereof having protease activity or the amino acid sequence SEQ ID NO 2.

**32.** A kit according to any of the claims 30 to 31,
**characterized in that**
the amino acid sequence of the protease according to any of the claims 30 to 3 is encoded by the nucleic acid sequence SEQ ID NO 3, a part thereof or a degenerated version of the nucleic acid sequence SEQ ID NO 3.

**33.** Kit according to claim 32,
**characterized in that**
the material with a silica surface is a glass.

**34.** Kit according to any of the claims 30 to 33,
**characterized in that**
the material with an affinity to nucleic acids is a composition comprising magnetic glass particles.

**35.** Kit according to any of the claims 30 to 34,
**characterized in that**
the kit additionally comprises a lysis buffer, a washing buffer and an elution buffer.

**36.** An aequeous composition of a protease which is at least 80 % identical to the amino acid sequence SEQ ID NO 1, a proteolytical derivative thereof having protease activity or the amino acid sequence SEQ ID NO 2,
**characterized in that**
the composition comprises 10 mM Tris acetate, 5 mM calcium chloride, 5 mM calcium acetate, 1 mM EDTA, 50 % (V/V) Glycerin with a pH value of 5.5.

**37.** A composition according to claim 36,
**characterized in that**
the amino acid sequence of the protease is identical to the amino acid sequence SEQ ID NO 1, a proteolytical derivative thereof having protease activity or the amino acid sequence SEQ ID NO 2.

**38.** A composition according to any of the claims 36 to 37,
**characterized in that**
the amino acid sequence of the protease according to any of the claims 36 to 37 is encoded by the nucleic acid sequence SEQ ID NO 3, a part thereof or a degenerated version of the nucleic acid sequence SEQ ID NO 3.

**39.** Use of a kit according to any of the claims 30 to 35 or a composition according to the claims 36 to 38 for the purification of nucleic acids.

**40.** Use of a kit according to any of the claims 30 to 35 or a composition according to the claims 36 to 38 in diagnostics.

**Patentansprüche**

**1.** Verfahren zur Analyse einer Nicht-Protein-Zielkomponente eines aus einer biologischen Probe stammenden Gemischs aus Nicht-Protein- und Protein-Komponenten, wobei man in einem Verfahrensschritt das Gemisch mit einer eine Aminosäuresequenz mit wenigstens 80% Identität mit der Aminosäuresequenz SEQ ID NO 1, einem proteolytischen Derivat davon mit Proteaseaktivität oder der Aminosäuresequenz SEQ ID NO 2 aufweisenden Protease inkubiert.

**2.** Verfahren nach Anspruch 1,
**dadurch gekennzeichnet, daß**
die Aminosäuresequenz der Protease mit der Aminosäuresequenz SEQ ID NO 1, einem proteolytischen Derivat davon mit Proteaseaktivität oder der Aminosäuresequenz SEQ ID NO 2 identisch ist.

**3.** Verfahren nach einem der Ansprüche 1 bis 2,
**dadurch gekennzeichnet, daß**
die Aminosäuresequenz der Protease nach einem der Ansprüche 1 bis 2 von der Nukleinsäuresequenz SEQ ID NO 3, einem Teil davon oder einer degenerierten Version der Nukleinsäuresequenz SEQ ID NO 3 codiert ist.

**4.** Verfahren nach einem der Ansprüche 1 bis 3,
**dadurch gekennzeichnet, daß**
es sich bei der biologischen Probe um eine Flüssigkeit aus dem menschlichen oder tierischen Körper handelt.

**5.** Verfahren nach einem der Ansprüche 1 bis 4,
**dadurch gekennzeichnet, daß**
es sich bei der biologischen Probe um Blut, Blutplasma, Blutserum oder Urin handelt.

**6.** Verfahren nach einem der Ansprüche 1 bis 5,
**dadurch gekennzeichnet, daß**
die biologische Probe Bakterienzellen, eukaryontische Zellen, Viren oder Gemische davon umfaßt.

**7.** Verfahren nach einem der Ansprüche 1 bis 6,
**dadurch gekennzeichnet, daß**

die Nicht-Protein-Zielkomponente nach dem Inkubationsschritt an ein Material mit einer Affinität dafür gebunden, gegebenenfalls gewaschen und gegebenenfalls von dem Material mit einer Affinität dafür freigesetzt wird.

**8.** Verfahren nach einem der Ansprüche 1 bis 7,
**dadurch gekennzeichnet, daß**
das Gemisch aus Nicht-Protein- und Protein-Komponenten Nukleinsäuren umfaßt.

**9.** Verfahren nach Anspruch 8,
**dadurch gekennzeichnet, daß**
die Nukleinsäuren DNA oder/und RNA umfassen.

**10.** Verfahren zur Analyse einer Nukleinsäure-Zielkomponente eines Gemischs aus Nukleinsäuren umfassenden Nicht-Protein-Komponenten und Protein-Komponenten, wobei das Gemisch aus einer biologischen Probe stammt, wobei man in den Verfahrensschritten

a) das Gemisch mit einer eine Aminosäuresequenz mit wenigstens 80% Identität mit der Aminosäuresequenz SEQ ID NO 1, einem proteolytischen Derivat davon mit Proteaseaktivität oder der Aminosäuresequenz SEQ ID NO 2 aufweisenden Protease inkubiert,
b) die Nukleinsäure-Zielkomponente gegebenenfalls amplifiziert und
c) die Nukleinsäure-Zielkomponente bestimmt oder nachweist.

**11.** Verfahren nach Anspruch 10,
**dadurch gekennzeichnet, daß**
die Aminosäuresequenz der Protease mit der Aminosäuresequenz SEQ ID NO 1, einem proteolytischen Derivat davon mit Proteaseaktivität oder der Aminosäuresequenz SEQ ID NO 2 identisch ist.

**12.** Verfahren nach einem der Ansprüche 10 bis 11,
**dadurch gekennzeichnet, daß**
die Aminosäuresequenz der Protease von der Nukleinsäuresequenz SEQ ID NO 3, einem Teil davon oder einer degenerierten Version der Nukleinsäuresequenz SEQ ID NO 3 codiert ist.

**13.** Verfahren nach einem der Ansprüche 10 bis 12,
**dadurch gekennzeichnet, daß**
es sich bei der biologischen Probe um eine Flüssigkeit aus dem menschlichen oder tierischen Körper handelt.

**14.** Verfahren nach einem der Ansprüche 10 bis 13,
**dadurch gekennzeichnet, daß**
es sich bei der biologischen Probe um Blut, Blutplasma, Blutserum oder Urin handelt.

**15.** Verfahren nach einem der Ansprüche 10 bis 14,
**dadurch gekennzeichnet, daß**
die Nukleinsäuren DNA oder/und RNA umfassen.

**16.** Verfahren nach Anspruch 15,
**dadurch gekennzeichnet, daß**
die DNA oder/und die RNA aus einem Virus oder einem Mikroorganismus stammt.

**17.** Verfahren nach Anspruch 16,
**dadurch gekennzeichnet, daß**
es sich bei dem Virus um das Hepatitis-B-Virus, das Hepatitis-C-Virus oder das menschliche Immunschwächevirus handelt.

**18.** Verfahren nach einem der Ansprüche 10 bis 17,
**dadurch gekennzeichnet, daß**
die Nukleinsäure-Zielkomponente mit der Polymerasekettenreaktion amplifiziert wird.

**19.** Verfahren nach einem der Ansprüche 10 bis 18,
**dadurch gekennzeichnet, daß**

die Nukleinsäuren und die Nukleinsäure-Zielkomponente nach Schritt a) an ein Material mit einer Affinität für Nukleinsäuren gebunden, gegebenenfalls gewaschen und gegebenenfalls von dem Material mit einer Affinität für Nukleinsäuren freigesetzt werden.

**20.** Verfahren nach Anspruch 19,
**dadurch gekennzeichnet, daß**
das Material mit einer Affinität für Nukleinsäuren und die Nukleinsäure-Zielkomponente ein Material mit einer Siliciumdioxidoberfläche umfaßt.

**21.** Verfahren nach Anspruch 20,
**dadurch gekennzeichnet, daß**
es sich bei dem Material mit einer Siliciumdioxidoberfläche um ein Glas handelt.

**22.** Verfahren nach einem der Ansprüche 19 bis 20,
**dadurch gekennzeichnet, daß**
es sich bei dem Material mit einer Affinität für Nukleinsäuren und der Nukleinsäure-Zielkomponente um eine magnetische Glaspartikel umfassende Zusammensetzung handelt.

**23.** Verwendung des Verfahrens nach einem der Ansprüche 1 bis 22 für die diagnostische Analyse.

**24.** Verwendung einer Protease mit der in einem der Ansprüche 10 bis 12 angegebenen Bedeutung in der Diagnostik.

**25.** Verwendung einer Protease mit der in einem der Ansprüche 10 bis 12 angegebenen Bedeutung zur Analyse einer Nicht-Protein-Zielkomponente eines aus einer biologischen Probe stammenden Gemischs aus Nicht-Protein- und Protein-Komponenten.

**26.** Verwendung einer Protease mit der in einem der Ansprüche 10 bis 12 angegebenen Bedeutung zur Anreicherung einer Nicht-Protein-Zielkomponente eines aus einer biologischen Probe stammenden Gemischs aus Nicht-Protein- und Protein-Komponenten.

**27.** Verwendung einer Protease mit der in einem der Ansprüche 10 bis 12 angegebenen Bedeutung zur Aufreinigung oder Isolierung einer Nicht-Protein-Zielkomponente eines aus einer biologischen Probe stammenden Gemischs aus Nicht-Protein- und Protein-Komponenten.

**28.** Verwendung nach einem der Ansprüche 25 bis 27,
**dadurch gekennzeichnet, daß**
es sich bei der Nicht-Protein-Zielkomponente um eine Nukleinsäure handelt.

**29.** Verwendung nach Anspruch 28,
**dadurch gekennzeichnet, daß**
die Nukleinsäure aus einem Virus oder einem Mikroorganismus stammt.

**30.** Kit-of-parts,
**dadurch gekennzeichnet, daß**
darin eine eine Aminosäuresequenz mit wenigstens 80% Identität mit der Aminosäuresequenz SEQ ID NO 1, einem proteolytischen Derivat davon mit Proteaseaktivität oder der Aminosäuresequenz SEQ ID NO 2 aufweisende Protease sowie ein ein Material mit einer Siliciumdioxidoberfläche umfassendes Material mit einer Affinität für Nukleinsäuren enthalten ist.

**31.** Kit nach Anspruch 30,
**dadurch gekennzeichnet, daß**
die Aminosäuresequenz der Protease mit der Aminosäuresequenz SEQ ID NO 1, einem proteolytischen Derivat davon mit Proteaseaktivität oder der Aminosäuresequenz SEQ ID NO 2 identisch ist.

**32.** Kit nach einem der Ansprüche 30 bis 31,
**dadurch gekennzeichnet, daß**
die Aminosäuresequenz der Protease nach einem der Ansprüche 30 bis 31 von der Nukleinsäuresequenz SEQ ID NO 3, einem Teil davon oder einer degenerierten Version der Nukleinsäuresequenz SEQ ID NO 3 codiert ist.

**33.** Kit nach Anspruch 32,
**dadurch gekennzeichnet, daß**
es sich bei dem Material mit einer Siliciumdioxidoberfläche um ein Glas handelt.

**34.** Kit nach einem der Ansprüche 30 bis 33,
**dadurch gekennzeichnet, daß**
es sich bei dem Material mit einer Affinität für Nukleinsäuren um eine magnetische Glaspartikel umfassende Zusammensetzung handelt.

**35.** Kit nach einem der Ansprüche 30 bis 34,
**dadurch gekennzeichnet, daß**
der Kit zusätzlich einen Lysepuffer, einen Waschpuffer und einen Elutionspuffer umfaßt.

**36.** Wässrige Zusammensetzung einer Protease mit wenigstens 80% Identität mit der Aminosäuresequenz SEQ ID NO 1, einem proteolytischen Derivat davon mit Proteaseaktivität oder der Aminosäuresequenz SEQ ID NO 2,
**dadurch gekennzeichnet, daß**
die Zusammensetzung 10 mM Tris-Acetat, 5 mM Calciumchlorid, 5 mM Calciumacetat, 1mM EDTA, 50 Vol.-% Glycerin bei einem pH-Wert von 5,5 umfaßt.

**37.** Zusammensetzung nach Anspruch 36,
**dadurch gekennzeichnet, daß**
die Aminosäuresequenz der Protease mit der Aminosäuresequenz SEQ ID NO 1, einem proteolytischen Derivat davon mit Proteaseaktivität oder der Aminosäuresequenz SEQ ID NO 2 identisch ist.

**38.** Zusammensetzung nach einem der Ansprüche 36 bis 37,
**dadurch gekennzeichnet, daß**
die Aminosäuresequenz der Protease nach einem der Ansprüche 36 bis 37 von der Nukleinsäuresequenz SEQ ID NO 3, einem Teil davon oder einer degenerierten Version der Nukleinsäuresequenz SEQ ID NO 3 codiert ist.

**39.** Verwendung eines Kits nach einem der Ansprüche 30 bis 35 oder einer Zusammensetzung nach Anspruch 36 bis 38 zur Aufreinigung von Nukleinsäuren.

**40.** Verwendung eines Kits nach einem der Ansprüche 30 bis 35 oder einer Zusammensetzung nach Anspruch 36 bis 38 in der Diagnostik.

## Revendications

**1.** Procédé pour l'analyse d'un composant cible non protéinique d'un mélange de composants non protéiniques et protéiniques dérivé d'un échantillon biologique comprenant l'étape d'incubation du mélange avec une protéase ayant une séquence d'acides aminés qui est identique au moins à 80 % à la séquence d'acides aminés SEQ ID NO 1, à un dérivé protéolytique de celle-ci ayant une activité protéase ou à la séquence d'acides aminés SEQ ID NO 2.

**2.** Procédé selon la revendication 1,
**caractérisé en ce que**
la séquence d'acides aminés de la protéase est identique à la séquence d'acides aminés SEQ ID NO 1, à un dérivé protéolytique de celle-ci ayant une activité protéase ou à la séquence d'acides aminés SEQ ID NO 2.

**3.** Procédé selon l'une quelconque des revendications 1 à 2,
**caractérisé en ce que**
la séquence d'acides aminés de la protéase selon l'une quelconque des revendications 1 à 2 est codée par la séquence d'acide nucléique SEQ ID NO 3, par une partie de celle-ci ou par une version dégénérée de la séquence d'acide nucléique SEQ ID NO 3.

**4.** Procédé selon l'une quelconque des revendications 1 à 3,
**caractérisé en ce que**
l'échantillon biologique est un liquide provenant du corps humain ou animal.

5. Procédé selon l'une quelconque des revendications 1 à 4, **caractérisé en ce que** l'échantillon biologique est du sang, du plasma sanguin, du sérum sanguin ou de l'urine.

6. Procédé selon l'une quelconque des revendications 1 à 5, **caractérisé en ce que** l'échantillon biologique comprend des cellules bactériennes, des cellules eucaryotiques, des virus ou des mélanges de ceux-ci.

7. Procédé selon l'une quelconque des revendications 1 à 6, **caractérisé en ce que** après l'étape d'incubation, le composant cible non protéinique est lié à un matériau ayant une affinité pour lui, puis est éventuellement lavé et éventuellement libéré du matériau ayant une affinité pour lui.

8. Procédé selon l'une quelconque des revendications 1 à 7, **caractérisé en ce que** le mélange de composants non protéiniques et protéiniques comprend des acides nucléiques.

9. Procédé selon la revendication 8, **caractérisé en ce que** les acides nucléiques comprennent de l'ADN ou de l'ARN ou les deux.

10. Procédé pour l'analyse d'un composant d'acide nucléique cible d'un mélange de composants non protéiniques, qui comprennent des acides nucléiques, et de composants protéiniques, le mélange étant dérivé d'un échantillon biologique, le procédé comprenant les étapes :

    a) d'incubation du mélange avec une protéase ayant une séquence d'acides aminés qui est identique au moins à 80 % à la séquence d'acides aminés SEQ ID NO 1, à un dérivé protéolytique de celle-ci ayant une activité protéase ou à la séquence d'acides aminés SEQ ID NO 2,
    b) éventuellement, d'amplification du composant d'acide nucléique cible, et
    c) de détermination ou de détection du composant d'acide nucléique cible.

11. Procédé selon la revendication 10, **caractérisé en ce que** la séquence d'acides aminés de la protéase est identique à la séquence d'acides aminés SEQ ID NO 1, à un dérivé protéolytique de celle-ci ayant une activité protéase ou à la séquence d'acides aminés SEQ ID NO 2.

12. Procédé selon l'une quelconque des revendications 10 à 11, **caractérisé en ce que** la séquence d'acides aminés de la protéase est codée par la séquence d'acide nucléique SEQ ID NO 3, par une partie de celle-ci ou par une version dégénérée de la séquence d'acide nucléique SEQ ID NO 3.

13. Procédé selon l'une quelconque des revendications 10 à 12, **caractérisé en ce que** l'échantillon biologique est un liquide provenant du corps humain ou animal.

14. Procédé selon l'une quelconque des revendications 10 à 13, **caractérisé en ce que** l'échantillon biologique est du sang, du plasma sanguin, du sérum sanguin ou de l'urine.

15. Procédé selon l'une quelconque des revendications 10 à 14, **caractérisé en ce que** les acides nucléiques comprennent de l'ADN ou de l'ARN ou les deux.

16. Procédé selon la revendication 15, **caractérisé en ce que** l'ADN ou l'ARN ou les deux sont dérivés d'un virus ou d'un microorganisme.

**17.** Procédé selon la revendication 16,
**caractérisé en ce que**
le virus est le virus de l'hépatite B, le virus de l'hépatite C ou le virus de l'immunodéficience humaine.

**18.** Procédé selon l'une quelconque des revendications 10 à 17,
**caractérisé en ce que**
le composant d'acide nucléique cible est amplifié avec la réaction d'amplification en chaîne par polymérase.

**19.** Procédé selon l'une quelconque des revendications 10 à 18,
**caractérisé en ce que**
après l'étape a) les acides nucléiques et le composant d'acide nucléique cible sont liés à un matériau ayant une affinité pour les acides nucléiques, puis sont éventuellement lavés et éventuellement libérés du matériau ayant une affinité pour les acides nucléiques.

**20.** Procédé selon la revendication 19,
**caractérisé en ce que**
le matériau ayant une affinité pour les acides nucléiques et le composant d'acide nucléique cible comprennent un matériau avec une surface de silice.

**21.** Procédé selon la revendication 20,
**caractérisé en ce que**
le matériau avec une surface de silice est un verre.

**22.** Procédé selon l'une quelconque des revendications 19 à 20,
**caractérisé en ce que**
le matériau ayant une affinité pour les acides nucléiques et le composant d'acide nucléique cible est une composition comprenant des particules de verre magnétique.

**23.** Utilisation du procédé selon l'une quelconque des revendications 1 à 22 pour l'analyse diagnostique.

**24.** Utilisation d'une protéase telle que spécifiée dans l'une quelconque des revendications 10 à 12 en diagnostic.

**25.** Utilisation d'une protéase telle que spécifiée dans l'une quelconque des revendications 10 à 12 pour l'analyse d'un composant non protéinique cible d'un mélange de composants non protéiniques et protéiniques dérivé d'un échantillon biologique.

**26.** Utilisation d'une protéase telle que spécifiée dans l'une quelconque des revendications 10 à 12 pour l'enrichissement d'un composant non protéinique cible d'un mélange de composants non protéiniques et protéiniques dérivé d'un échantillon biologique.

**27.** Utilisation d'une protéase telle que spécifiée dans l'une quelconque des revendications 10 à 12 pour la purification ou l'isolement d'un composant non protéinique cible d'un mélange de composants non protéiniques et protéiniques dérivé d'un échantillon biologique.

**28.** Utilisation selon l'une quelconque des revendications 25 à 27,
**caractérisée en ce que**
le composant non protéinique cible est un acide nucléique.

**29.** Utilisation selon la revendication 28,
**caractérisée en ce que**
l'acide nucléique provient d'un virus ou d'un microorganisme.

**30.** Trousse d'éléments,
**caractérisée en ce que**
elle contient une protéase ayant une séquence d'acides aminés, qui est identique au moins à 80 % à la séquence d'acides aminés SEQ ID NO 1, à un dérivé protéolytique de celle-ci ayant une activité protéase ou à la séquence d'acides aminés SEQ ID NO 2, et un matériau ayant une affinité pour les acides nucléiques qui comprend un matériau avec une surface de silice.

**31.** Trousse selon la revendication 30,
**caractérisée en ce que**
la séquence d'acides aminés de la protéase est identique à la séquence d'acides aminés SEQ ID NO 1, à un dérivé protéolytique de celle-ci ayant une activité protéase ou à la séquence d'acides aminés SEQ ID NO 2.

**32.** Trousse selon l'une quelconque des revendications 30 à 31,
**caractérisée en ce que**
la séquence d'acides aminés de la protéase selon l'une quelconque des revendications 30 à 31 est codée par la séquence d'acide nucléique SEQ ID NO 3, par une partie de celle-ci ou par une version dégénérée de la séquence d'acide nucléique SEQ ID NO 3.

**33.** Trousse selon la revendication 32,
**caractérisée en ce que**
le matériau avec une surface de silice est un verre.

**34.** Trousse selon l'une quelconque des revendications 30 à 33,
**caractérisée en ce que**
le matériau ayant une affinité pour les acides nucléiques est une composition comprenant des particules de verre magnétique.

**35.** Trousse selon l'une quelconque des revendications 30 à 34,
**caractérisée en ce que**
la trousse comprend en outre un tampon de lyse, un tampon de lavage et un tampon d'élution.

**36.** Composition aqueuse d'une protéase qui est identique au moins à 80 % à la séquence d'acides aminés SEQ ID NO 1, à un dérivé protéolytique de celle-ci ayant une activité protéase ou à la séquence d'acides aminés SEQ ID NO 2,
**caractérisée en ce que**
la composition comprend du Tris acétate 10 mM, du chlorure de calcium 5 mM, de l'acétate de calcium 5 mM, de l'EDTA 1 mM, de la glycérine à 50 % (V/V) avec une valeur de pH de 5,5.

**37.** Composition selon la revendication 36,
**caractérisée en ce que**
la séquence d'acides aminés de la protéase est identique à la séquence d'acides aminés SEQ ID NO 1, à un dérivé protéolytique de celle-ci ayant une activité protéase ou à la séquence d'acides aminés SEQ ID NO 2.

**38.** Composition selon l'une quelconque des revendications 36 à 37,
**caractérisée en ce que**
la séquence d'acides aminés de la protéase selon l'une quelconque des revendications 36 à 37 est codée par la séquence d'acide nucléique SEQ ID NO 3, par une partie de celle-ci ou par une version dégénérée de la séquence d'acide nucléique SEQ ID NO 3.

**39.** Utilisation d'une trousse selon l'une quelconque des revendications 30 à 35 ou d'une composition selon les revendications 36 à 38 pour la purification d'acides nucléiques.

**40.** Utilisation d'une trousse selon l'une quelconque des revendications 30 à 35 ou d'une composition selon les revendications 36 à 38 en diagnostic.

Figure 1a

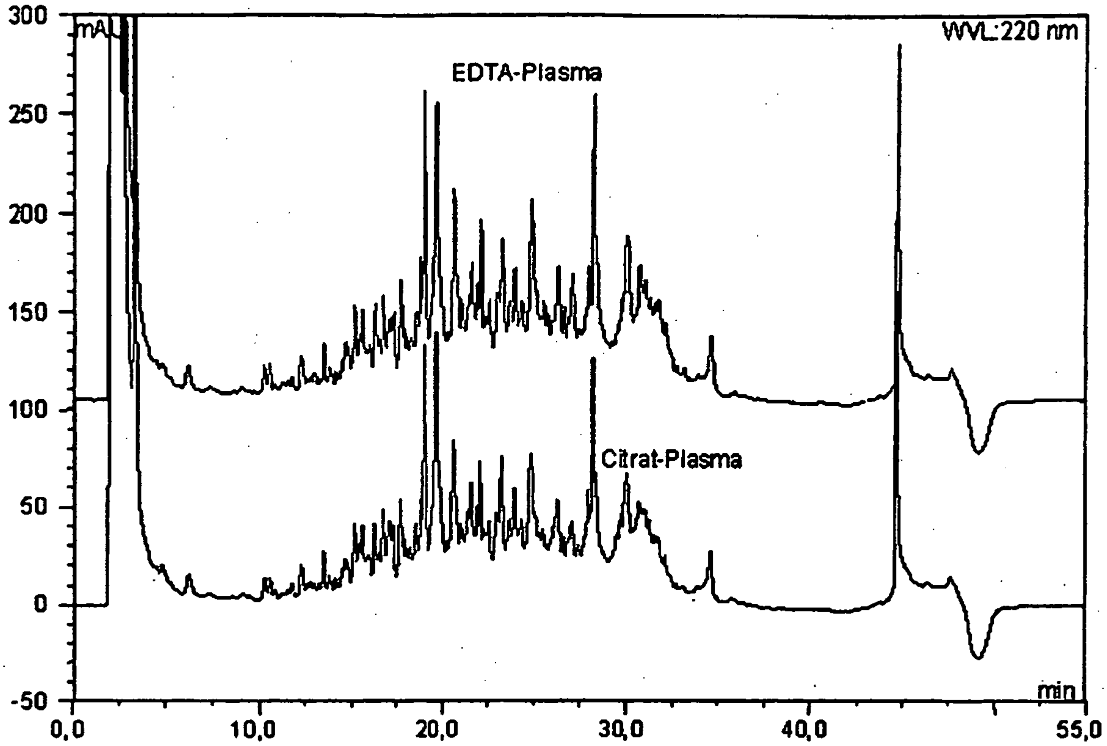
300
250
200
150
100
50
0
-50
mAU
WVL:220 nm
EDTA-Plasma
Citrat-Plasma
min
0,0
10,0
20,0
30,0
40,0
55,0

Figure 1b)

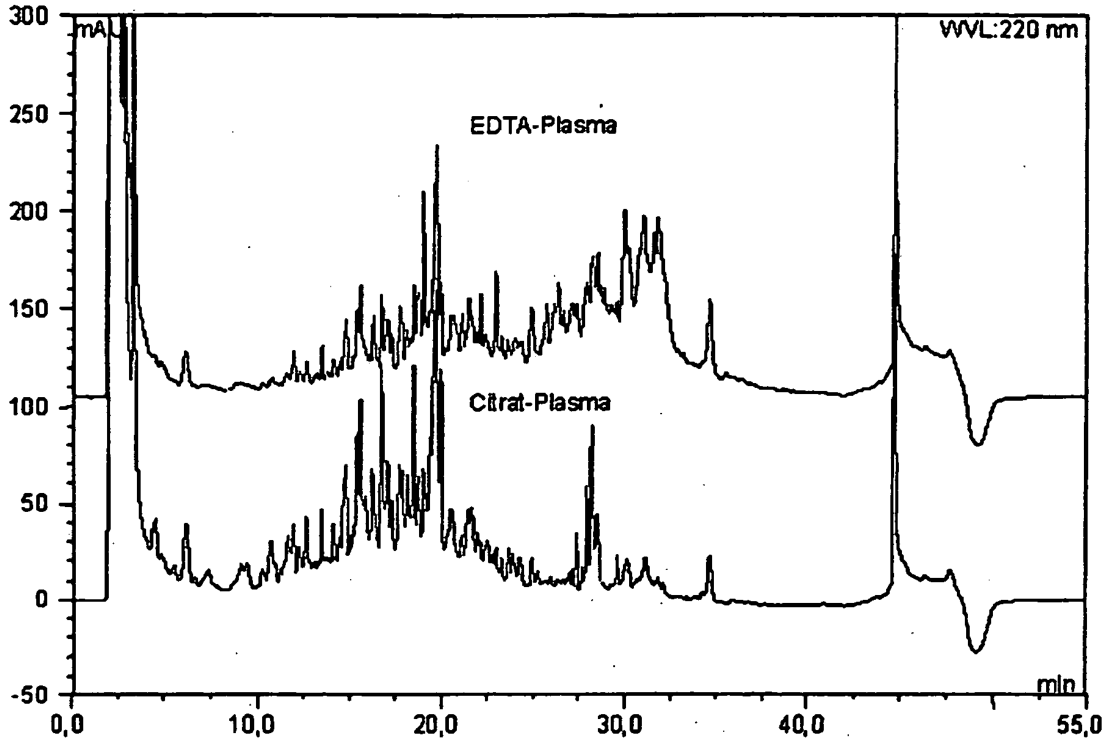
300
250
200
150
100
50
0
-50
mAU
WVL:220 nm
EDTA-Plasma
Citrat-Plasma
min
0,0
10,0
20,0
30,0
40,0
55,0

Figure 2

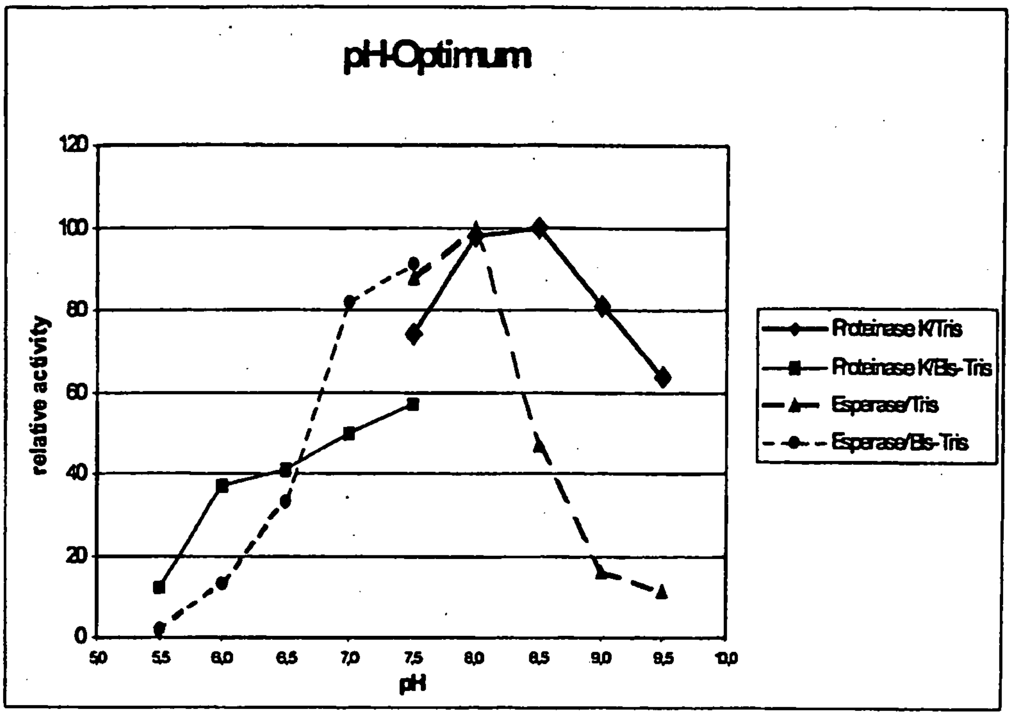
pH-Optimum
relative activity
0
20
40
60
80
100
120
5,0
5,5
6,0
6,5
7,0
7,5
8,0
8,5
9,0
9,5
10,0
pH
Proteinase K/Tris
Proteinase K/Bis-Tris
Esperase/Tris
Esperase/Bis-Tris

Figure 3

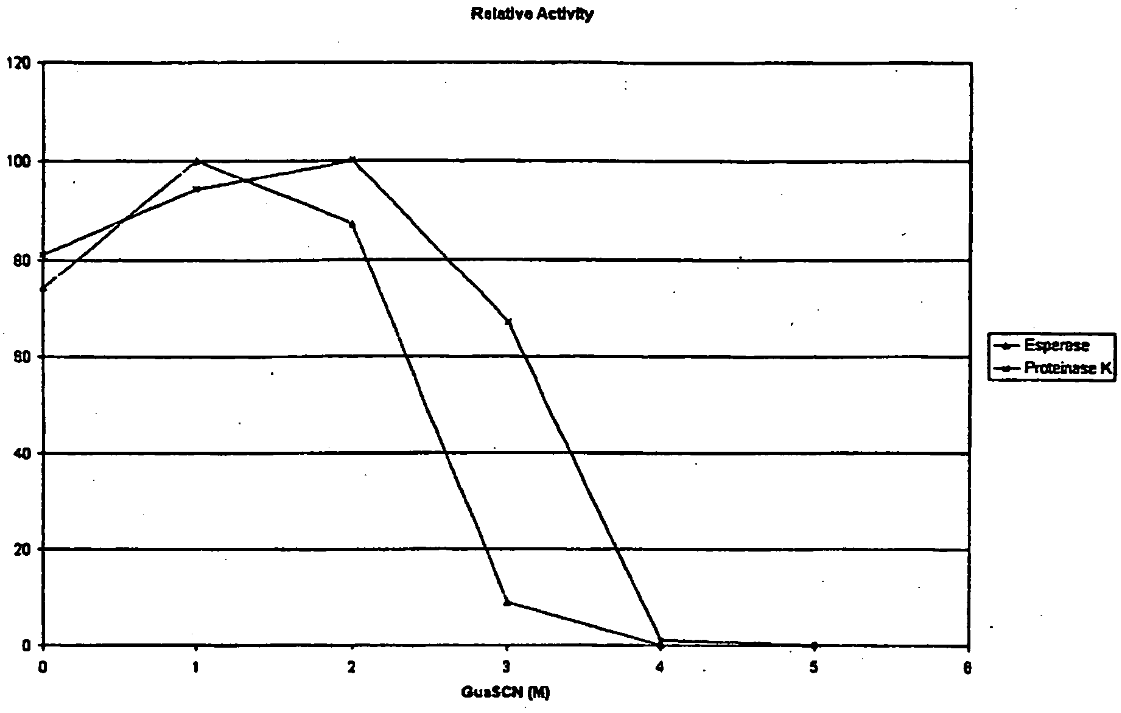
Relative Activity
120
100
80
60
40
20
0
0
1
2
3
4
5
6
GuaSCN (M)
Esperase
Proteinase K

Figure 4

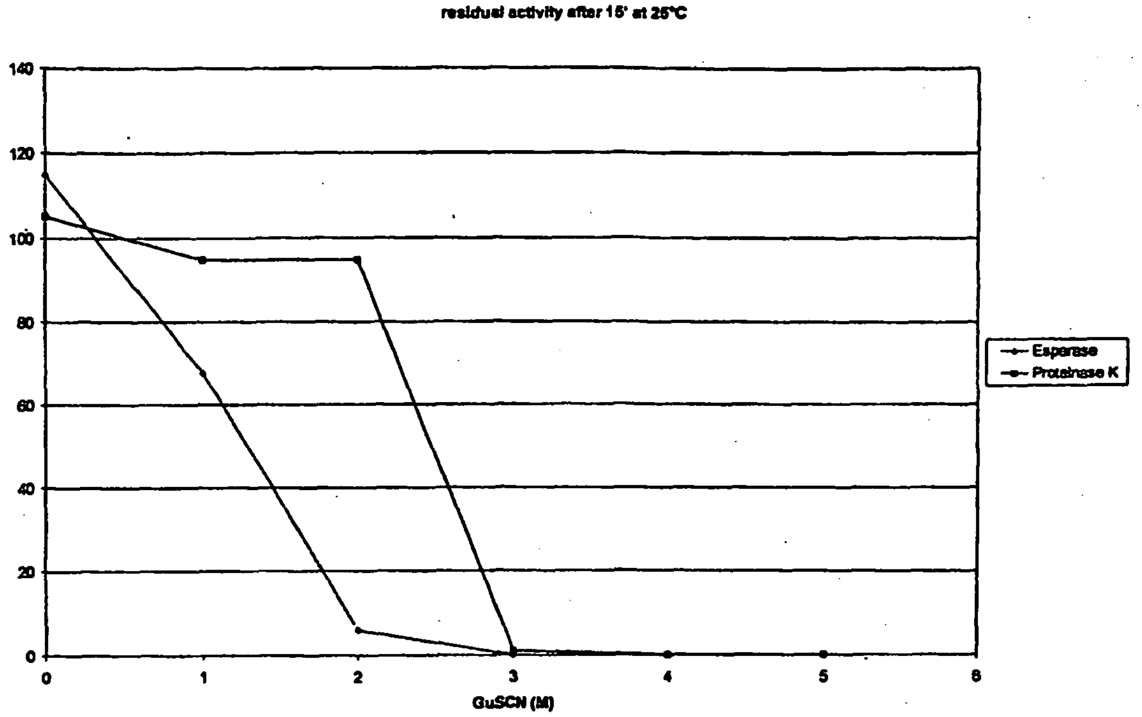
residual activity after 15' at 25°C
140
120
100
80
60
40
20
0
0
1
2
3
4
5
6
GuSCN (M)
Esperase
Proteinase K

Figure 5

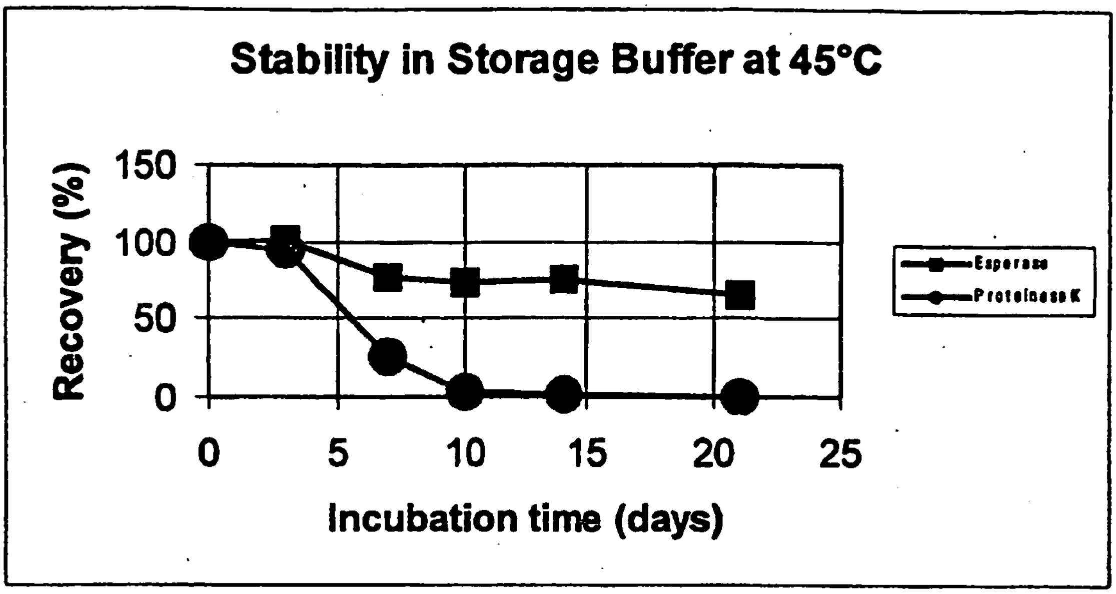
Stability in Storage Buffer at 45°C
Recovery (%)
150
100
50
0
0
5
10
15
20
25
Incubation time (days)
Esperase
Proteinase K

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4683195 A
- WO 9804730 A
- US 5386024 A
- WO 8906279 A
- US 3723250 A
- EP 396608 A
- US 5741694 A
- WO 9820115 A
- DE 3734442 A
- GB 9100212 A
- WO 0137291 A
- DE 3724442
- WO 9001069 A
- EP 439182 A2
- WO 920880 A
- US 5130238 A
- WO 9202638 A
- US 5210015 A
- US 5804375 A
- US 5487972 A
- WO 9916781 A
- EP 110165 A
- EP 439182 A


### Non-patent literature cited in the description


- **WALSH.** Enzymatic Reaction Mechanisms. W. H. Freeman and Company, 1979
- **WHITE ; HANDLER ; SMITH.** Principles of Biochemistry. McGraw-Hill Book Company, 1973, 271-272
- **PRIEST.** *Bacteriological Rev.,* 1977, vol. 41, 711-753
- **KURIHARA et al.** *J. Biol. Chem.,* 1972, vol. 247, 5629-5631
- **STAHL ; FERRARI.** *J. Bacteriol.,* 1984, vol. 158, 411-418
- **VASANTHA et al.** *J. Bacteriol.,* 1984, vol. 159, 811-819
- **JACOBS et al.** *Nucl. Acids Res.,* 1985, vol. 13, 8913-8926
- **NEDKOV et al.** *Biol. Chem. Hoppe-Seyler,* 1985, vol. 366, 421-430
- **SVENDSEN et al.** *FEBS Lett,* 1986, vol. 196, 228-232
- **MELOUN et al.** *FEBS. Lett.,* 1985, vol. 183, 195-200
- **JANY ; MAYER.** *Biol. Chem. Hoppe-Seyler,* 1985, vol. 366, 584-492
- **KRAUT.** *Ann. Rev. Biochem.,* 1977, vol. 46, 331-358
- **SVENDSEN et al.** *FEBS Lett.,* 1986, vol. 196, 228-232
- **MELOUN et al.** *FEBS Lett.,* 1985, vol. 183, 195-200
- **JANY ; MAYER.** *Biol. Chem. Hoppe-Seyler,* 1985, vol. 366, 485-492
- **SAMBROOK et al.** Molecular Cloning. Cold Spring Harbor University Press, 1989
- Bioanalytik. Spektrum Akademischer Verlag, 1998
- **SAMBROOK et al.** Molecular Cloning, A Laboratory Manual. Cold Spring Harbour Laboratory Press
- **AUSUBEL et al.** Current Protocols in Molecular Biology. J. Wiley and Sons, 1987
- *Proc. Natl. Acad. USA,* 1979, vol. 76, 615-691
- *Anal. Biochem.,* 1982, vol. 121, 382-387
- *Anal. Biochem.,* 1988, vol. 175, 196-201
- *Anal. Biochem.,* 1992, vol. 201, 166-169
- *Analytical Biochemistry,* 1988, vol. 175, 196-201
- **WU ; WALLACE.** *Genomics,* 1989, vol. 4, 560-569
- **BARANY.** *Proc. Natl. Acad. Sci. USA,* 1991, vol. 88, 189-193
- **BARANY.** *PCR Methods and Applic.,* 1991, vol. 1, 5-16
- **KWOH et al.** *Proc. Natl. Acad. Sci. USA,* 1989, vol. 86, 1173-1177
- **GUATELLI et al.** *Proc. Natl. Acad. Sci. USA,* 1990, vol. 87, 1874-1878
- **WHELEN ; PERSING.** *Annu. Rev. Microbiol.,* 1996, vol. 50, 349-373

- **ABRAMSON ; MYERS.** *Current Opinion in Biotechnology,* 1993, vol. 4, 41-47
- **WALLACE.** *Genomics,* 1989, vol. 4, 560-569
- **WALSH.** Enzymatic Reaction Mechanisms. W. H. Freeman and Company, 1979

SEQUENCE LISTING

<110> Roche Diagnostics GmbH

<120> Methods for the analysis of non-proteinaceous components using a protease from a Bacillus strain

<130> Esperase

<140>
<141>

<160> 12

<170> PatentIn Ver. 2.1

<210> 1
<211> 361
<212> PRT
<213> Bacillus lentus

<300>
<310> WO89/06279
<311> 1989-01-06
<312> 1989-07-13

<300>
<310> EP396608
<311> 1989-01-06
<312> 1990-11-14

<400> 1

Met Arg Gln Ser Leu Lys Val Met Val Leu Ser Thr Val Ala Leu Leu
1 5 10 15

Phe Met Ala Asn Pro Ala Ala Ala Gly Gly Glu Lys Lys Glu Tyr Leu
20 25 30

Ile Val Val Glu Pro Glu Glu Val Ser Ala Gln Ser Val Glu Glu Ser
35 40 45

Tyr Asp Val Asp Val Ile His Glu Phe Glu Glu Ile Pro Val Ile His
50 55 60

Ala Glu Leu Thr Lys Lys Glu Leu Lys Lys Leu Lys Lys Asp Pro Asn
65 70 75 80

Val Lys Ala Ile Glu Glu Asn Ala Glu Val Thr Ile Ser Gln Thr Val
85 90 95

Pro Trp Gly Ile Ser Phe Ile Asn Thr Gln Gln Ala His Asn Arg Gly
100 105 110

Ile Phe Gly Asn Gly Ala Arg Val Ala Val Leu Asp Thr Gly Ile Ala
115 120 125

Ser His Pro Asp Leu Arg Ile Ala Gly Gly Ala Ser Phe Ile Ser Ser
130 135 140

Glu Pro Ser Tyr His Asp Asn Asn Gly His Gly Thr His Val Ala Gly
145 150 155 160

Thr Ile Ala Ala Leu Asn Asn Ser Ile Gly Val Leu Gly Val Arg Pro
165 170 175

Ser Ala Asp Leu Tyr Ala Leu Lys Val Leu Asp Arg Asn Gly Ser Gly
180 185 190

Ser Leu Ala Ser Val Ala Gln Gly Ile Glu Trp Ala Ile Asn Asn Asn
195 200 205

Met His Ile Ile Asn Met Ser Leu Gly Ser Thr Ser Gly Ser Ser Thr
210 215 220

Leu Glu Leu Ala Val Asn Arg Ala Asn Asn Ala Gly Ile Leu Leu Val
225 230 235 240

Gly Ala Ala Gly Asn Thr Gly Arg Gln Gly Val Asn Tyr Pro Ala Arg
245 250 255

Tyr Ser Gly Val Met Ala Val Ala Ala Val Asp Gln Asn Gly Gln Arg
260 265 270

Ala Ser Phe Ser Thr Tyr Gly Pro Glu Ile Glu Ile Ser Ala Pro Gly
275 280 285

Val Asn Val Asn Ser Thr Tyr Thr Gly Asn Arg Tyr Val Ser Leu Ser
290 295 300

Gly Thr Ser Met Ala Thr Pro His Val Ala Gly Val Ala Ala Leu Val
305 310 315 320

Lys Ser Arg Tyr Pro Ser Tyr Thr Asn Asn Gln Ile Arg Gln Arg Ile
325 330 335

Asn Gln Thr Ala Thr Tyr Leu Gly Ser Pro Ser Leu Tyr Gly Asn Gly
340 345 350

Leu Val His Ala Gly Arg Ala Thr Gln
355 360

<210> 2
<211> 268
<212> PRT
<213> Bacillus lentus

<400> 2

Gln Thr Val Pro Trp Gly Ile Ser Phe Ile Asn Thr Gln Gln Ala His
1 5 10 15

Asn Arg Gly Ile Phe Gly Asn Gly Ala Arg Val Ala Val Leu Asp Thr
20 25 30

Gly Ile Ala Ser His Pro Asp Leu Arg Ile Ala Gly Gly Ala Ser Phe
35 40 45

Ile Ser Ser Glu Pro Ser Tyr His Asp Asn Asn Gly His Gly Thr His
50 55 60

Val Ala Gly Thr Ile Ala Ala Leu Asn Asn Ser Ile Gly Val Leu Gly
65 70 75 80

Val Arg Pro Ser Ala Asp Leu Tyr Ala Leu Lys Val Leu Asp Arg Asn
85 90 95

Gly Ser Gly Ser Leu Ala Ser Val Ala Gln Gly Ile Glu Trp Ala Ile
100 105 110

Asn Asn Asn Met His Ile Ile Asn Met Ser Leu Gly Ser Thr Ser Gly
115 120 125

Ser Ser Thr Leu Glu Leu Ala Val Asn Arg Ala Asn Asn Ala Gly Ile
130 135 140

Leu Leu Val Gly Ala Ala Gly Asn Thr Gly Arg Gln Gly Val Asn Tyr
145 150 155 160

Pro Ala Arg Tyr Ser Gly Val Met Ala Val Ala Ala Val Asp Gln Asn
165 170 175

Gly Gln Arg Ala Ser Phe Ser Thr Tyr Gly Pro Glu Ile Glu Ile Ser
180 185 190

Ala Pro Gly Val Asn Val Asn Ser Thr Tyr Thr Gly Asn Arg Tyr Val
195 200 205

Ser Leu Ser Gly Thr Ser Met Ala Thr Pro His Val Ala Gly Val Ala
210 215 220

Ala Leu Val Lys Ser Arg Tyr Pro Ser Tyr Thr Asn Asn Gln Ile Arg
225 230 235 240

Gln Arg Ile Asn Gln Thr Ala Thr Tyr Leu Gly Ser Pro Ser Leu Tyr
245 250 255

Gly Asn Gly Leu Val His Ala Gly Arg Ala Thr Gln
260 265

<210> 3
<211> 1086
<212> DNA
<213> Bacillus lentus

<400> 3

atgagacaaa gtctaaaagt tatggttttg tcaacagtgg cattgctttt catggcaaac 60
ccagcagcag caggcgggga gaaaaaggaa tatttgattg tcgtcgaacc tgaagaagtt 120
tctgctcaga gtgtcgaaga aagttatgat gtggacgtca tccatgaatt tgaagagatt 180
ccagtcattc atgcagaact aactaaaaaa gaattgaaaa aattaaagaa agatccgaac 240
gtaaaagcca tcgaagagaa tgcagaagta accatcagtc aaacggttcc ttggggaatt 300
tcattcatta atacgcagca agcgcacaac cgcggtattt ttggtaacgg tgctcgagtc 360
gctgtccttg atacaggaat tgcttcacac ccagacttac gaattgcagg gggagcgagc 420
tttatttcaa gcgagccttc ctatcatgac aataacggac acggaactca cgtggctggt 480
acaatcgctg cgttaaacaa ttcaatcggt gtgcttggtg tacgaccatc ggctgacttg 540
tacgctctca aagttcttga tcggaatgga agtggttcgc ttgcttctgt agctcaagga 600
atcgaatggg caattaacaa caacatgcac attattaata tgagccttgg aagcacgagt 660
ggttctagca cgttagagtt agctgtcaac cgagcaaaca atgctggtat tctcttagta 720
ggggcagcag gtaatacggg tagacaagga gttaactatc ctgctagata ctctggtgtt 780
atggcggttg cagcagttga tcaaaatggt caacgcgcaa gcttctctac gtatggccca 840
gaaattgaaa tttctgcacc tggtgtcaac gtaaacagca cgtacacagg caatcgttac 900
gtatcgcttt ctggaacatc tatggcaaca ccacacgttg ctggagttgc tgcacttgtg 960
aagagcagat atcctagcta tacgaacaac caaattcgcc agcgtattaa tcaaacagca 1020
acgtatctag gttctcctag cctttatggc aatggattag tacatgctgg acgtgcaaca 1080
caataa 1086

<210> 4
<211> 24
<212> DNA
<213> Hepatitis C virus

<400> 4
gcagaaagcg tctagccatg gcgt 24

<210> 5
<211> 24
<212> DNA
<213> Hepatitis C virus

<220>
<221> modified_base
<222> (1)
<223> Biotin derivatization

<400> 5
ctcgcaagca ccctatcagg cagt 24

<210> 6
<211> 21
<212> DNA
<213> Hepatitis C virus

<220>
<221> modified_base
<222> (1)
<223> Ruthenium3+-(tris-bipyridyl)-derivatisation

<400> 6
gtcgtgcagc ctccaggacc c 21

<210> 7
<211> 30
<212> DNA
<213> Human immunodeficiency virus

<220>
<221> modified_base
<222> (1)
<223> Biotin derivatization

<400> 7
agttggagga catcaagcag ccatgcaaat 30

<210> 8
<211> 27
<212> DNA
<213> Human immunodeficiency virus

<220>
<221> modified_base
<222> (1)
<223> Biotin derivatization

<400> 8
tgctatgtca gttccccttg gttctct 27

<210> 9
<211> 20
<212> DNA
<213> Human immunodeficiency virus

<220>
<221> modified_base
<222> (1)
<223> Ruthenium3+-(tris-bipyridyl)-derivatisation

<400> 9
atcaatgagg aagctgcaga 20

<210> 10
<211> 18
<212> DNA
<213> Hepatitis B virus

<400> 10
ggagtgtgga ttcgcact 18

<210> 11
<211> 18
<212> DNA

<213> Hepatitis B virus

<220>
<221> modified_base
<222> (1)
<223> Biotin derivatization

<400> 11
tgagatcttc tgcgacgc 18

<210> 12
<211> 18
<212> DNA
<213> Hepatitis B virus

<220>
<221> modified_base
<222> (1)
<223> Ruthenium3+-(tris-bipyridyl)-derivatisation

<400> 12
agaccaccaa atgcccct 18